# EUROPEAN PATENT APPLICATION

(11) **EP 4 357 446 A1**
(43) Date of publication of application: **24.04.2024**
(21) Application number: 22824636.9
(22) Date of filing: 30.03.2022
(51) Int. Cl.: C12M 1/34, C12Q 1/686, C12Q 1/6869, G06Q 50/04, G01N 27/62

(54) **STATE INFERENCE SYSTEM AND STATE INFERENCE METHOD**

(30) Priority: 18.06.2021 JP 2021102070
(71) Applicant: Epistra Inc., Tokyo 105-0013 (JP)
(72) Inventor: OZAWA, Yosuke, Tokyo 105-0013 (JP); TSUZUKI, Taku, Tokyo 105-0013 (JP)
(74) Representative: Gille Hrabal Partnerschaftsgesellschaft mbB Patentanwälte
(86) International application number: PCT/JP2022/016221
(87) International publication number: WO 2022/264640

(57) **Abstract**

Provided are a state inference system and a state inference method, which can improve the efficiency of bioproduction as well as research and development using a biological sample. A state inference system 1a is provided with a measurement unit 2 for measuring characteristic values of a plurality of culture media and at least one target culture medium; and a state inference unit 10a for inferring a culture state of a culture from characteristic values of the target culture medium on the basis of a feature amount extraction model that characterizes a distribution of characteristic values of the plurality of culture media. In the state inference system 1a, since it can be inferred in advance whether a desired culture state will be obtained in bioproduction as well as research and development using a target culture medium, the time required for making a proper decision on the target culture medium can be shortened. As a result, the efficiency of bioproduction or research and development using a culture medium can be improved.

## Description

### Technical Field

The present invention relates to a state inference system and a state inference method.

### Background Art

Biologically derived raw materials such as natural culture media are widely used as culture media, samples, reagents, and the like that are used for cell culture; however, it is known that biologically derived raw materials have lot-to-lot variation or batch-to-batch variation. This becomes a direct cause of variations in the quality of cell culture, and in the processes of bioproduction, research and development, and examination, a phenomenon often occurs in which even when the processes are executed under exactly identical conditions, the execution results vary greatly.

Analyzing the state of a culture medium before carrying out cell culture by using the culture medium, is important for increasing the efficiency of bioproduction or research and development using the culture medium, and for example, as shown in PTL 1, a method of analyzing components of a culture medium by using a spectroscopic analysis method has been disclosed.

### Citation List

### Patent Literature

PTL 1: JP2013-544353A

### Summary of Invention

### Technical Problem

However, only by simply measuring the components of a culture medium by using a spectroscopic analysis method, it is difficult to decide in advance from the measurement result whether a desired result of bioproduction or research and development can be finally obtained by using a biological sample such as a culture medium, and it is necessary to actually carry out bioproduction or research and development using a biological sample to the end and check whether the biological sample is a biological sample that will finally give a desired result. Therefore, since it requires time to decide whether a biological sample is a biological sample that will give a desired result in bioproduction as well as research and development using a biological sample, it is desirable to shorten the time required for making the decision and improve the efficiency of bioproduction or research and development using a biological sample.

Thus, it is an object of the invention to provide a state inference system and a state inference method, which can improve the efficiency of bioproduction as well as research and development using a biological sample.

### Solution to Problem

A state inference system according to the invention includes: a measurement unit for using any of a culture medium, a culture cultured by using the culture medium, a culture medium extract extracted from the culture medium, and a culture extract extracted from the culture, as a biological sample and a target biological sample, and measuring characteristics of a plurality of biological samples including the biological sample at a certain time point and characteristic of the target biological sample being the same type as the biological sample at the certain time point; a database for storing a feature amount extraction model that characterizes a distribution of input data including at least characteristic values of the plurality of biological samples acquired through the measurement unit; and a state inference unit for inferring a culture state of the culture or inferring a culture time during which the culture reaches a predetermined culture state, from the input data of the target biological sample on the basis of the feature amount extraction model.

A state inference method according to the invention includes: a measurement step of using any of a culture medium, a culture cultured by using the culture medium, a culture medium extract extracted from the culture medium, and a culture extract extracted from the culture, as a biological sample and a target biological sample, and measuring characteristics of a plurality of biological samples including the biological sample at a certain time point and characteristic of the target biological sample being the same type as the biological sample at the certain time point, in a measurement unit; a storing step of storing, in a database, a feature amount extraction model that characterizes a distribution of input data including at least characteristic values of the plurality of biological samples acquired through the measurement unit; and a state inference step of inferring a culture state of the culture by using a state inference unit or inferring a culture time during which the culture reaches a predetermined culture state by using a state inference unit, from the input data of the target biological sample on the basis of the feature amount extraction model.

### Advantageous Effects of Invention

According to the invention, since it can be inferred in advance whether a desired culture state can be obtained in bioproduction as well as research and development using a target biological sample, the time required for making a proper decision of a target biological sample may be shortened by using these inference results as a guide. As a result, the efficiency of bioproduction or research and development using a biological sample can be improved.

### Brief Description of Drawings

[FIG. 1] FIG. 1 is a block diagram illustrating a configuration of a state inference system.
[FIG. 2] FIG. 2 is a schematic view for describing a measurement time for characteristics of a culture medium and a measurement time for a culture state of a culture.
[FIG. 3] FIG. 3 is graphs showing examples of mass spectra obtained from a mass spectrometer and examples of measurement vectors produced from the mass spectra.
[FIG. 4] FIG. 4 is a table showing culture results when Escherichia coli was cultured by using each sample.
[FIG. 5] FIG. 5 is a table showing culture results when Escherichia coli was cultured by using other samples, and state inference results predicted by the state inference system.
[FIG. 6] FIG. 6 is a graph showing a relationship between the actual culture results and the state inference results.
[FIG. 7] FIG. 7 is graphs showing measurement vectors of additives.
[FIG. 8] FIG. 8 is a table showing inner products of the measurement vectors of additives and a principal component vector.
[FIG. 9] FIG. 9 is a table showing the ranges of improvement brought by each additive.
[FIG. 10] FIG. 10 is a schematic view for describing another embodiment related to the measurement time for characteristics of a culture medium.
[FIG. 11] FIG. 11 is a schematic view for describing another embodiment related to the measurement time for a culture state of a culture.
[FIG. 12] FIG. 12 is a block diagram illustrating a configuration (2) of another state inference system.
[FIG. 13] FIG. 13 is a schematic view for describing an example in which culture A is induced to differentiate into culture B, culture C, or non-intended target cells by culturing.
[FIG. 14] FIG. 14 is a block diagram illustrating a configuration of a state inference system according to a fourth embodiment.
[FIG. 15] FIG. 15 is a schematic view for describing a method for selecting culture A in a sorting unit.
[FIG. 16] 16A of FIG. 16 is a schematic view for describing an example in which culture A is induced to differentiate into culture E or non-intended target cells by culturing, and 16B of FIG. 16 is a schematic view for describing an example in which culture A is induced to differentiate into culture D or non-intended target cells by culturing, and culture D is further induced to differentiate into culture E or non-intended target cells by culturing.

### Description of Embodiments

An embodiment of the invention will be described in detail below with reference to the drawings. In the following description, identical constituent elements will be assigned with identical reference numerals, and any redundant description will not be repeated herein.

### (1) First Embodiment

### (1-1) Configuration of State Inference System

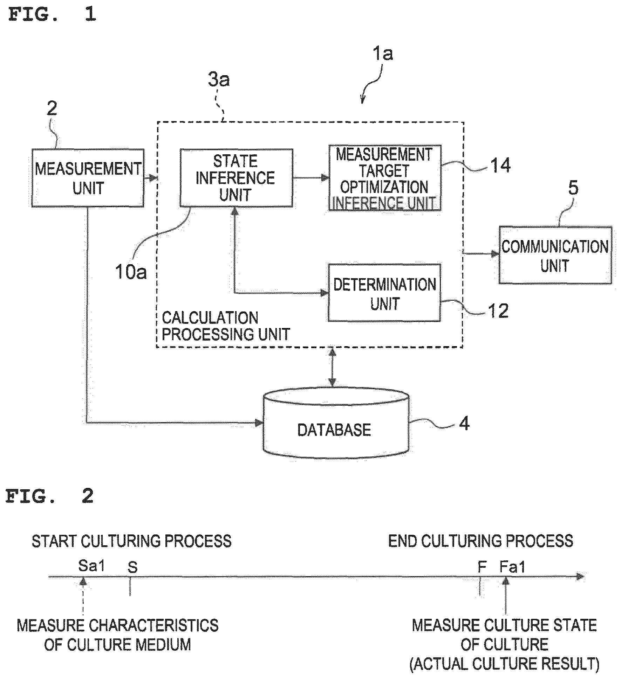

FIG. 1 is a block diagram illustrating a configuration of a state inference system 1a according to the present embodiment. In the first embodiment, an example of a culture medium will be described as an example of a biological sample. In addition, the first embodiment is an example of applying a mass spectrometer as a measurement unit 2 that will be described below. The state inference system 1a according to the present embodiment infers the state of a culture medium used for a cell culture experiment or the culture state of a culture on the basis of a feature amount extraction model specifying a feature amount, and has the measurement unit 2, a calculation processing unit 3a, a database 4, and a communication unit 5.

The term "culture state" according to the present embodiment indicates, for example, a state of the yield of a culture per unit volume of the culture medium or a state of the quality of the culture, which are included in the culture medium used for a culturing process. The yield of a culture also includes the yield of a culture extract extracted from a cultured culture, and the yield of a component in the culture medium secreted by a cultured culture. Regarding the yield of a culture extract, for example, in a case where the culture is Escherichia coli, the yield of a useful protein (for example, an enzyme) extracted from Escherichia coli, and the like may be mentioned. Regarding the yield of a component in the culture medium secreted by a cultured culture, for example, in a case where the culture is CHO cells, the yield of a biopharmaceutical (for example, an antibody) extracted from the culture medium, and the like may be mentioned. In the present embodiment, the state of the yield of a culture per unit volume of the culture medium, which is included in the culture medium used for a culturing process, will be mainly described as the culture state.

The quality of a culture indicates the functionality of the culture, and for example, in a case where the culture is cells, the quality of the culture corresponds to an indication of how much therapeutic effect (function) the cells that are being cultured will have on an intended disease, and the like as an indicator of quality, an indication of the state of differentiation of the cells as an indicator of quality, or the like. In addition, the quality of the culture as a culture state of a biological sample also includes the quality of a culture extract extracted from a cultured culture, and the quality of a component in the culture medium secreted by a cultured culture. The quality of a culture extract is, for example, in a case where the culture is Escherichia coli, an indication of how good (being functional) is the reaction efficiency of a useful protein (for example, an enzyme) extracted from Escherichia coli, as an indicator of quality, or the like. The quality of a component in the culture medium secreted by a culture corresponds to, for example, in a case where the culture is CHO cells, an indication of how much a biopharmaceutical (for example, an antibody) extracted from the culture medium will have an intended function, and the like as an indicator of quality.

The measurement unit 2 according to the present embodiment is a mass spectrometer, and a mass spectrum is obtained by subjecting the culture medium to mass spectrometry. In the present embodiment, a case of applying a mass spectrometer as the measurement unit is described; however, the invention is not limited to this, and for example, an impedance sensor, a microscope apparatus, a Raman spectrometer, chromatography, a digital PCR measurement apparatus, a nuclear magnetic resonance apparatus, an antibody quantification kit, or a nucleic acid sequencing apparatus may be applied as the measurement unit. Examples in which the impedance sensor, microscope apparatus, and the like are applied as the measurement unit will be described below.

Here, in the mass spectrometer as the measurement unit 2, a culture medium obtained by mixing a matrix, a solvent, and the like is irradiated with laser light to generate ions, the ions are detected by using an ion detector, and a mass spectrum is generated on the basis of a detection signal from the ion detector.

As the mass spectrometer, for example, a mass spectrometer using an ion source, such as an Electron Ionization (EI) method, a Chemical Ionization (CI) method, a Fast Atom Bombardment (FAB) method, a Matrix Assisted Laser Desorption Ionization (MALDI) method, or an Electro Spray Ionization (ESI) method, may also be applied. In addition, as the mass spectrometer, a mass spectrometer using a mass sorting unit (analyzer), such as a magnetic field sector type, quadrupole type, ion trap type, or time-of-flight type mass sorting unit; a mass spectrometer using an ion detector such as an electron multiplier or a microchannel plate, and the like may be applied.

According to the present specification, the term "culture medium" is a culture medium for culturing a culture, the culture medium including nutrients used for the growth of cells, microorganisms, or viruses (hereinafter, simply referred to as culture) and providing a growth environment for the culture. Regarding the culture medium according to the present embodiment, it is possible to use a known culture medium that is used for culturing various cultures, and the culture medium is appropriately determined according to the type of the culture. For example, as the culture medium, culture media obtained by incorporating inorganic salts, carbohydrates, amino acids, vitamins, proteins, peptides, fatty acids, lipids, blood serum, and the like into buffer solutions may be mentioned.

Here, the "cells" are not particularly limited as long as they can be cultured using a culture medium, and examples include insect cells, animal cells, human cells, and plant cells, while other examples include myocardial cells, neural stem cells, mesenchymal stem cells, embryonic stem cells, hepatic cells, corneal stem cells, pancreatic islet cells, tumor cells, iPS cells, ES cells, and blood cells. Specifically, 293T cells may be mentioned. The "cells" also include cellular tissues, which are aggregates of cells, and for example, artificial meat, retinal tissue, cardiac muscle sheets, and multicellular biological tissues (for example, fetuses and plants) are also included. Examples of the "microorganisms" include bacteria, fungi, microalgae, protozoa, and yeast. Examples of the "bacteria" include Escherichia coli, archaebacteria (for example, Thermus thermophilus), Bacillus subtilis, and cyanobacteria.

In the present specification, a culture medium for each lot or each batch, which may have a possibility of having variations in the components due to the lot-to-lot variation, batch-to-batch variation, or the like, is each referred to as a single culture medium. When there are a plurality of such culture media, that is, culture media prepared from a plurality of different lots, or culture media prepared from a plurality of different batches, are referred to as "a plurality of culture media".

The state inference system 1a according to the present embodiment performs, for example, mass spectrometry of a plurality of culture media by using the measurement unit 2 and generates a feature amount extraction model on the basis of the obtained mass spectrometry data. Here, the mass spectrometry data represent a mass spectrum measured when a culture medium is subjected to mass spectrometry by the measurement unit 2, or a measurement vector (will be described below) generated from the mass spectrum, and the mass spectrometry data may serve as characteristic values of the culture medium. The state inference system 1a can thereafter perform mass spectrometry using the measurement unit 2 on a culture medium whose components are unknown, and on the basis of the mass spectrometry data obtained as a result and the above-described feature amount extraction model, the state inference system 1a can infer the culture state of the culture when a culture medium whose components are unknown is used. In this way, a biological sample with which the culture state of a culture is inferred by using the generated feature amount extraction model, is referred to as "target biological sample". In the present embodiment, a case of applying a culture medium as an example of the biological sample will be described below, and a culture medium with which the culture state of a culture is inferred by using the generated feature amount extraction model, is referred to as "target culture medium".

The measurement unit 2 performs mass spectrometry on each of a plurality of culture media and outputs the obtained mass spectra to the calculation processing unit 3a and the database 4. When the mass spectra (mass spectrometry data) of the plurality of culture media are inputted from the measurement unit 2, the database 4 stores the mass spectrum of each culture medium in association with the date and time when the mass spectrum was measured. In the database 4, information on the background of the culture medium (the background includes, for example, the composition of the culture medium (specification information), product number, lot number, date of manufacture, date of arrival, and past usage history) is stored, and furthermore, information on parameters related to the culturing conditions (hereinafter, also referred to as culture parameters), such as the culture temperature and culture time of the culturing process, the pH of the culture medium, and the stirring speed for the culture medium, is also stored in association with the mass spectrometry data. Furthermore, the database 4 also stores various data such as the feature amount extraction model generated by the calculation processing unit 3a.

The calculation processing unit 3a has a state inference unit 10a, a determination unit 12, and a measurement target optimization inference unit 14. The state inference unit 10a receives mass spectra of a plurality of culture media inputted from the measurement unit 2 or the database 4.

In the state inference unit 10a according to the present embodiment, a feature amount extraction model is generated by, for example, performing multivariate analysis by using mass spectrometry data of the plurality of culture media obtained through the measurement unit 2 at a certain time point, or a result of the culture state (hereinafter, also referred to as a culture result) of a culture when the culture is actually cultured by using the culture media. With such a feature amount extraction model, the components of a culture medium affecting the culture result when a culture is cultured by using the culture media can be analyzed.

Regarding the feature amount extraction model, in the case of supervised learning, it is desirable to apply a regression model or the like, which utilizes mass spectrometry data of a target culture medium as the input, and an inference result on the future culture state (hereinafter, also referred to as a state inference result) that is finally obtained when it is assumed that culturing of a culture is continued as it is by using the target culture medium, as the output. In addition, when the feature amount extraction model is generated by supervised learning, it is desirable to allow the feature amount extraction model to learn the correspondence relation between the mass spectrometry data of a plurality of culture media and the culture result obtained when these culture media are used, and characterize the distribution of the mass spectrometry data from the culture result.

Regarding supervised learning of other feature amount extraction models, for example, the mass spectrometry data of a plurality of culture media, each culture result obtained when these culture media are used, and a truth label indicating whether the culture result is a desired result (for example, an accuracy indicating whether the culture result is a desired culture result) are used, and when the mass spectrometry data of the culture media and the culture result are learned, this learning may be carried out by associating the mass spectrometry data and the culture result with the truth label, so that the distribution of the mass spectrometry data may be characterized.

As the feature amount extraction model, for example, various feature amount extraction models such as principal component analysis (PCA), partial least squares (PLS), polynomial regression, Gaussian process regression, and random forest regression can be applied. As the feature amount extraction model, for example, a machine learning model generated by machine learning of the characteristic values (mass spectrometry data) of the culture media and the culture result, may also be applied.

As the machine learning model, for example, a neural network (for example, a convolutional neural network (CNN) or a Bayesian neural network (BNN)) can be applied. In addition, the feature amount extraction model may involve supervised learning as described above, as well as unsupervised learning. The details in the case of generating the feature amount extraction model by unsupervised learning will be described below; however, when the feature amount extraction model is generated by unsupervised learning, it is desirable to use the mass spectrometry data of a plurality of culture media and allow learning that characterizes the distribution of a plurality of these mass spectrometry data.

The determination unit 12 determines the state of a target culture medium on the basis of the inference result for the culture state of the culture (state inference result) obtained by the state inference unit 10a. The determination unit 12 according to the present embodiment sets identification values that enable stepwise identification between a satisfactory culture medium and a poor culture medium, in a state inference space in which a principal component vector (feature amount) is defined for the feature amount extraction model, the mass spectrometry data of the target culture medium are projected onto the state inference space, and the state of the target culture medium is inferred by using the identification values as a guide (state determination processing).

Here, a culture medium in which the mass of a culture per unit volume of the culture medium finally obtained when the culture is cultured by using a culture medium becomes equal to or greater than a predetermined optimum set value (hereinafter, referred to as satisfactory determination value) set in advance, is referred to as "satisfactory culture medium", and a culture medium in which the mass becomes equal to or less than a predetermined set value lower than the satisfactory determination value (hereinafter, referred to as poor determination value) is referred to as "poor culture medium".

The determination unit 12 may set a threshold value for abnormality determination in the state inference space in which the principal component vector is defined for the feature amount extraction model (abnormality determination processing). As a result, when a state inference vector obtained by projecting the mass spectrometry data of the target culture medium onto the state inference space does not exceed the threshold value, the determination unit 12 may determine that the target culture medium is a target culture medium that will give a satisfactory culture result, and when the state inference vector is less than the threshold value, the determination unit 12 may determine that the target culture medium is a target culture medium that will give a poor culture result.

In the present specification, a culture medium whose characteristics do not meet the required criteria due to the lot-to-lot variation, batch-to-batch variation, or the like in the culture medium, will be referred to as an abnormal culture medium, and a culture medium whose characteristics satisfy the required criteria will be referred to as a normal culture medium.

The measurement target optimization inference unit 14 infers an additive to be added to the target culture medium on the basis of the state inference result obtained in the state inference unit 10a, in order to obtain a target culture medium in which a desired culture state is obtained (measurement target optimization inference processing). As a result, even for a target culture medium which is inferred that a desired culture state cannot be obtained in the state inference unit 10a, an operator or the like can add an appropriate additive to this target culture medium to correct the target culture medium into a target culture medium in which a desired culture state is obtained.

When the measurement target optimization inference unit 14 infers what kind of additive should be added to the target culture medium in order to obtain a desired culture result, the measurement target optimization inference unit 14 outputs the obtained inference result on the additive to the communication unit 5 that will be described below. The inference result for the additive obtained in the measurement target optimization inference unit 14 is suggested to the operator or the like through the communication unit 5. As a result, the operator or the like may execute a culturing process in the target culture medium with enhanced performance by adding the additive (inferred additive) suggested by the state inference system 1a to the target culture medium, and can attempt to obtain a desired culture result.

Regarding the communication unit 5, for example, a display device that displays the state inference result, the determination result, and the like in the state inference unit 10a on a display unit to allow the operator or the like to visually recognize the results; a transmission device that transmits an e-mail to the operator or the like, and communicates the state inference result, the determination result, and the like; a sound emitting device that communicates the state inference result, the determination result, and the like to the operator or the like by voice; and the like can be applied. Examples of the operator or the like include a manipulator of the state inference system 1a, an experimenter of a culturing experiment, and an arbitrarily determined administrator.

Even for the feature amount extraction model generated by the calculation processing unit 3a, or the measurement results (mass spectra and measurement vectors) obtained by the measurement unit 2 and the result of an inferred additive obtained by the measurement target optimization inference unit 14, the communication unit 5 may make the contents thereof known to the operator or the like through a communication device, a transmission device, and the like.

Next, the state inference unit 10a, determination unit 12, and measurement target optimization inference unit 14 mentioned above will be described in order.

### (1-2) State Inference Unit

Next, the state inference processing in the state inference unit 10a will be described. Here, as an example, an example of generating a feature amount extraction model by PLS regression will be described. The culture state of a culture inferred by the state inference unit 10a may be the quality (functionality) of the culture obtained by a culturing process as described above; however, here, a case where the culture state is the yield of the culture obtained by a culturing process will be mainly described as an example.

Here, FIG. 2 is a schematic view showing the timing of measuring the mass spectrometry data of a plurality of culture media and the culture state of the culture, which are obtained upon generating a feature amount extraction model by the state inference unit 10a. S in FIG. 2 represents the start timing of a culturing process, and F represents the end timing of the culturing process after a predetermined culture period has elapsed. The "start timing S of a culturing process" means the time when culturing a culture using a culture medium under culturing conditions with predetermined culture parameters is started, and the "end timing F of a culturing process" means the time when culturing of a culture using the culture medium is ended. In the state inference system 1a according to the present embodiment, as shown in FIG. 2, the mass spectrometry data of a plurality of culture media are obtained through the measurement unit 2 before the start (before the start timing S) of the culturing process (Sa1). In the state inference system 1a, after the end (after end timing F) of the culturing process, the yield of the culture after actually culturing each culture by using each of the culture media is examined, and the culture state of the culture is measured (Fa1).

In the state inference unit 10a, the relevance between the mass spectrometry data of the culture medium before the start of the culturing process (before use in culturing) and the yield of the culture (that is, culture result) included in the culture medium after the end of the culturing process (after use in culturing) obtained in this way is characterized, and from mass spectrometry data measured for a target culture medium prior to use in culturing, whose components are unknown, the yield of the culture (culture state of the culture) that will be obtained in the future when the target culture medium is used in the culturing process, is inferred. As a result, whether the target culture medium is a target culture medium in which a desired culture state is obtainable, may be inferred before the culturing process is started.

In this way, it is important to infer in advance, before the culturing process is started, what kind of culture state the culture will reach when the target culture medium is used in a culturing process, in order to quickly detect the success or failure of the culturing process in a case where there are variations such as lot-to-lot variation in the initial culture medium, for example, as in the case of production of biopharmaceuticals and the like. In addition, by specifying in advance the causes of culture failure caused by the culture medium, and taking measures such as correcting the culture medium so as to obtain desired culture results according to the content of the causes, the yield and quality can be stabilized. Furthermore, since failing of culturing can be suppressed beforehand by utilizing the state inference result obtained with the state inference system 1a, extensive economic damage caused by failure of culturing can be prevented.

In the present embodiment, for example, an example of culturing Escherichia coli as a culture by using a culture medium to which yeast extract is added, will be described. With regard to such culturing of Escherichia coli, even when the same amount of yeast extract is added to the same culture medium, the growth performance of Escherichia coli may vary depending on the lot of yeast extract.

In this case, the state inference system 1a discovers the relevance between the mass spectrometry data of each of culture media to which different yeast extracts have been added, and the culture result obtained when Escherichia coli is cultured by using each of these culture media to which different yeast extracts have been added, and establishes a feature amount extraction model so that functional differences between these culture media can be clearly detected. In the state inference system 1a, the state of the yeast extract in the culture medium, which is related to the growth performance of Escherichia coli, can be inferred for a target culture medium whose components are unknown, by utilizing such a feature amount extraction model.

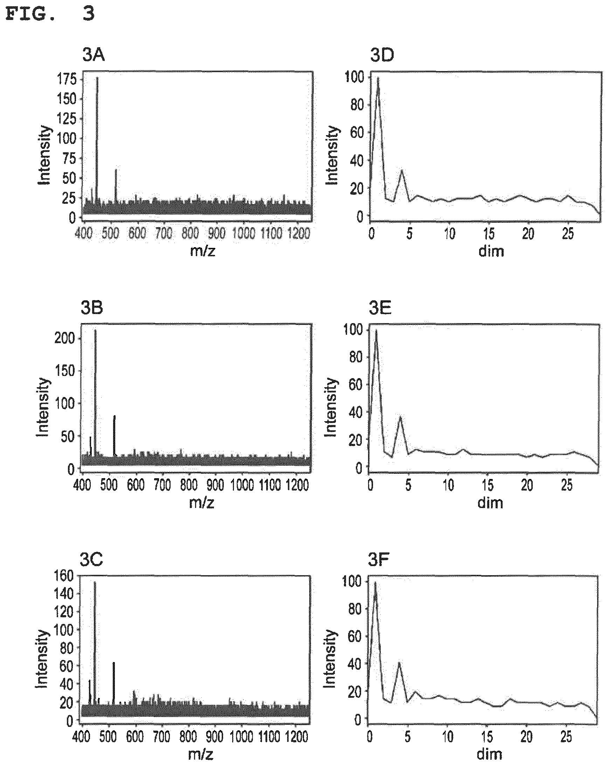

In the present embodiment, for example, mass spectrometry is performed by the measurement unit 2 for a plurality of types of aqueous solutions of yeast extract (culture media) from a plurality of different lots, and a mass spectrum of mass spectrometry regarding each aqueous solution of yeast extract is obtained. 3A, 3B, and 3C in FIG. 3 show examples of mass spectra obtained by subjecting each of different types of culture media (aqueous solutions of yeast extract) to mass spectrometry.

In 3A, 3B, and 3C in FIG. 3, the horizontal axis represents the m/z value, and the vertical axis represents the signal intensity. The m/z value on the horizontal axis is a measured value that is directly read out from primary measurement results of a mass spectrometer, and the m/z value is generally defined as "a dimensionless quantity obtained by dividing a dimensionless quantity, which is obtained by dividing the mass of an ion by a unified atomic mass unit (= dalton), further by the absolute value of the charge number of the ion" . The peak intensity in a mass spectrum corresponds to the amount of each ion having the m/z value, which is generated by ionization of an analyte (culture medium).

When the state inference unit 10a according to the present embodiment receives mass spectra obtained by mass spectrometry from the measurement unit 2 or the database 4, the state inference unit 10a performs standardization and coarse graining for the mass spectra and generates a measurement vector in which the amount of information on the signal intensity has been reduced. Specifically, as the standardization, each peak intensity is corrected such that the maximum peak intensity of the signal intensity in the mass spectrum becomes 100. In addition, as the coarse graining, the m/z value on the horizontal axis in the mass spectrum is divided into predetermined dimensions (here, 30 dimensions), the signal intensity is averaged for each dimension, and a measurement vector indicating the peak intensity with respect to the maximum peak intensity 100 for each dimension is obtained. In the present embodiment, the measurement vector obtained from the mass spectra is the mass spectrometry data and is used as a characteristic value of the target culture medium.

3D, 3E, and 3F in FIG. 3 show examples of the measurement vector, and 3D shows a measurement vector generated from the mass spectrum of 3A, 3E shows the measurement vector generated from the mass spectrum of 3B, while 3F shows the measurement vector generated from the mass spectrum of 3C.

3D, 3E, and 3F in FIG. 3 each represent an example in which the m/z value on the horizontal axis in the mass spectrum obtained by mass spectrometry is divided by a predetermined number of dimensions, the maximum peak intensity of the signal intensity is defined as 100 for each dimension, and the peak intensity with respect to the maximum peak intensity 100 is determined for each dimension by subtracting the maximum value and the minimum value within each dimension.

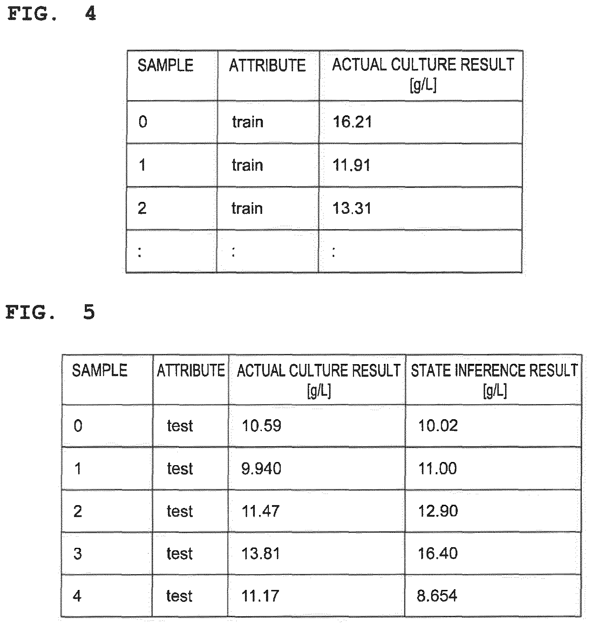

In the database 4, the measurement vector generated by the state inference unit 10a is also stored. In the database 4, for example, a culture result finally obtained when Escherichia coli as a culture is actually cultured by using a culture medium is stored as a culture result, as shown in FIG. 4. In FIG. 4, culture media with which the culture results are obtained are indicated as samples 0, 1, 2, and the like, and each of the actual culture results is shown for each culture medium. Furthermore, the term "train" shown in FIG. 4 means that the attributes of the data are sample data for generating a feature amount extraction model.

In this case, culturing of the same Escherichia coli is actually performed by using each of the culture media, Escherichia coli is collected from the culture fluid after the culturing of Escherichia coli, and the dry mass (hereinafter, also simply referred to as mass or yield) (g/L) of Escherichia coli per unit volume of the culture medium is measured to obtain a culture result. The culture result (actual state result) thus obtained when Escherichia coli is actually cultured by using each of the culture media, is stored in the database 4 in association with the measurement vector of each of the culture media.

The state inference unit 10a performs PLS regression by using the measurement vectors of a plurality of culture media (that is, being mass spectrometry data and characteristic values of the culture media) as explanatory variables, and using the mass (yield) (g/L) of Escherichia coli per unit volume of the culture medium, which is finally obtained as a culture result when Escherichia coli (culture) is cultured by using each of the culture media, as an objective variable. PLS regression is a technique of selecting a principal component vector (base) of a measurement vector so as to maximize the covariance between the substance content in the measurement vector and the yield of Escherichia coli.

As a result, the state inference unit 10a extracts a highly relevant multi-dimensional (for example, 30-dimensional) principal component vector (supervised base) to obtain a feature amount extraction model, for the purpose of obtaining a high yield of Escherichia coli per unit volume of the culture medium and a satisfactory culture result. In the feature amount extraction model, the component of a culture medium that is most contributed to the differences in the culture results can be specified on the basis of the obtained principal component vector.

Next, a case of inferring the state of yeast extract in a target culture medium whose components are unknown, by utilizing the feature amount extraction model that characterizes the distribution of the mass spectrometry data of culture media in this way, will be described. The state inference system 1a performs mass spectrometry by the measurement unit 2 before the start of the culturing process, for a target culture medium whose state is desired to be inferred and whose components are unknown, and a mass spectrum is obtained. The state inference unit 10a generates a measurement vector as the mass spectrometry data from the mass spectrum of the target culture medium whose state is desired to be inferred. The state inference unit 10a normalizes the obtained measurement vector with respect to the defined principal component vector and then projects the normalized measurement vector onto the state inference space of the feature amount extraction model, and thereby a state inference vector (hereinafter, also referred to as state inference result) regarding the target culture medium is obtained.

The value of the state inference vector, which is a state inference result, represents the mass of Escherichia coli per unit volume of the culture fluid (that is, culture state of the culture after the end of the culturing process), which is predicted when culturing is performed by using each yeast extract, and implies that as the state inference vector is further headed toward the positive direction, the yield of Escherichia coli expected as a culture result increases.

In this way, the state inference unit 10a can output an inference value of the yield of Escherichia coli (hereinafter, also referred to as state inference result), which is roughly assumed for a target culture medium whose components are unknown when Escherichia coli is cultured with the target culture medium in the future.

The state inference result obtained in the state inference unit 10a is stored in the database 4 and is also suggested to an operator or the like through the communication unit 5. In this way, in the state inference system 1a, the state inference result outputted by the state inference unit 10a can also be suggested directly to the operator or the like through the communication unit 5, without performing state determination for the target culture medium by the determination unit 12 that will be described below. Thus, in the state inference system 1a, an operator or the like can be allowed to infer whether the target culture medium is a target culture medium that will give a satisfactory culture result, before the target culture medium is actually used for culturing, on the basis of the state inference result obtained by inferring the culture state of a culture.

Here, FIG. 5 is a table showing: actual culture results (experimental results) obtained by actually performing culturing of the same Escherichia coli by using each of five types of target culture media (aqueous solutions of yeast extract) with different components, collecting Escherichia coli from the culture fluid after culturing of Escherichia coli, and measuring a dry mass (g/L) of Escherichia coli per unit volume of the culture medium; and state inference results (inferred yield results of Escherichia coli per unit volume of the culture medium included in the target culture medium after use in culturing) determined from each measurement vector of the five types of target culture media by using the feature amount extraction model generated in advance in the above-mentioned state inference system 1a.

In FIG. 5, the target culture media whose components are unknown are indicated as 0, 1, 2, 3, and 4. In addition, the term "test" shown in FIG. 5 means that the attribute of the data is sample data for analyzing the target culture media whose components are unknown, by using the generated feature amount extraction model.

As shown in FIG. 5, since the state inference results obtained by the state inference system 1a are close to the actual culture result, the operator or the like can be allowed to predict a culture result that will be obtained when the target culture media are used, by suggesting these state inference results to the operator or the like, without actually performing a culturing process using the target culture media.

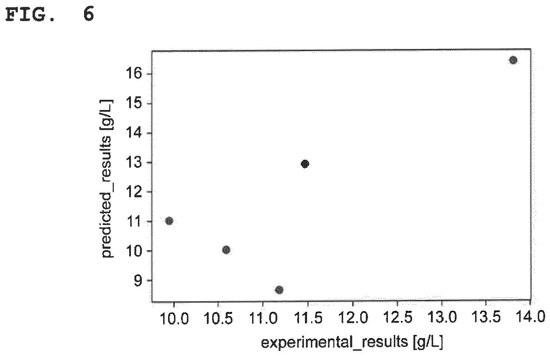

FIG. 6 is a graph showing the relationship between the state inference results shown in FIG. 5 and the actual culture results (actual state results) published for reference. The horizontal axis in FIG. 6 represents the actual culture result (actual state result), and the vertical axis represents the state inference result. Even from FIG. 6, it is shown that the state inference results obtained by the state inference system 1a can predict the actual culture results as a general tendency.

### (1-3) Determination Unit

Next, determination processing in the determination unit 12 will be described. The determination unit 12 determines the state of a target culture medium on the basis of the state inference result obtained by the state inference unit 10a.

Examples of the determination processing in the determination unit 12 include: state determination processing of simply determining stepwise whether the state of the target culture medium is in a state suitable for culturing a culture, without performing any abnormality determination that will be described below on the target culture medium before use in culturing; and abnormality determination processing of determining whether the target culture medium before use in culturing is in an abnormal state not suitable for culturing a culture.

In the case of performing state determination processing, the determination unit 12 sets identification values that enable stepwise identification between a satisfactory culture medium and a poor culture medium, in the state inference space in which the principal component vector (supervised base) is defined as a feature amount in the feature amount extraction model, prior to the state determination processing. In addition, these identification values may be associated with identification marks that indicate stepwise the degree of satisfactoriness regarding the state of a culture medium, for example, by taking the culture result obtained when the culture medium is used for culturing a culture, as a reference.

Regarding the setting of the identification values for the state inference space, any numerical values may be set by the operator or the like, or the principal component vector defined in the state inference space may keep being automatically divided into sections at predetermined intervals, and the identification values may be automatically set. The determination unit 12 determines the state of the target culture medium from the value of the state inference vector obtained by projecting the mass spectrometry data (measurement vector) of the target culture medium onto the state inference space, and from the identification values.

The determination unit 12 may also set a threshold value for abnormality determination in the state inference space for the feature amount extraction model, on the basis of a certain rule for the abnormality determination. In this case, setting of the threshold value may be set in the state inference space by the operator or the like in consideration of a desired culture result. For example, setting of the threshold value is achieved such that when not exceeding the threshold value, a satisfactory culture result equal to or higher than a reference value is obtained, and when exceeding the threshold value, a poor culture result less than a reference value is obtained.

The determination unit 12 determines the presence or absence of an abnormality of a target culture medium from the value of the state inference vector obtained by projecting the mass spectrometry data of the target culture medium onto the state inference space in which the principal component vector is defined, and from a threshold value. In addition, the degree of abnormality of the target culture medium may be determined by setting the above-described threshold value to be divided at a plurality of stages.

When a determination result for the target culture medium obtained by state determination processing is inputted, the communication unit 5 communicates the determination result obtained by determining the state of the target culture medium before use in culturing on the basis of the identification values, to the operator or the like. In addition, when the presence or absence of an abnormality based on abnormality determination processing or the determination result for the degree of abnormality is inputted, the communication unit 5 communicates the presence or absence of an abnormality or the degree of abnormality to the operator or the like.

For example, in a case where there is an abnormality, information indicating that there is an abnormality, including characteristic values of the target culture medium, error codes, and the like, may be communicated from the communication unit 5 to the operator or the like, and in a case where there is no abnormality, a notice that there is no abnormality may be communicated from the communication unit 5 to the operator or the like. In addition, the communication unit 5 may be set so as to perform communication to the operator or the like particularly only when there is an abnormality, or a display unit that lights up only when there is an abnormality may be provided to display only when there is an abnormality.

### (1-4) Measurement Target Optimization Inference Unit

Next, measurement target optimization inference processing in the measurement target optimization inference unit 14 will be described. When an inference result for the culture state (state inference result) of a culture is obtained in the state inference unit 10a from mass spectrometry data of a target culture medium before the start of a culturing process on the basis of the feature amount extraction model, the measurement target optimization inference unit 14 receives the state inference result from the state inference unit 10a. The measurement target optimization inference unit 14 analyzes the state inference result obtained in the state inference unit 10a and infers an additive that is included in the target culture medium after use in culturing and can maximize the yield of the culture per unit volume of the culture medium when added to the target culture medium before use in culturing. The measurement target optimization inference unit 14 allows the operator or the like to correct the components of the target culture medium to be used in the culturing process, by adding the additive that has been inferred (also referred to as inferred additive) to the target culture medium.

In the present embodiment, as the additive that corrects the components of the target culture medium, an additive that maximizes the yield of the culture per unit volume of the culture medium included in the target culture medium after use in culturing will be described as an example; however, the invention is not limited to this. For example, the additive may be an additive that improves the quality of the culture (for example, bringing the culture into a desired differentiated state) included in the target culture medium after use in culturing.

In this way, in the present embodiment, the composition of the target culture medium can be changed by adding an appropriate type of additive to the target culture medium, and the yield of the culture per unit volume of the culture medium (culture state) included in the target culture medium after use in culturing can be improved before the target culture medium is actually used in the culturing process.

Here, a case in which the culture state is improved by adding any one additive among three types of additives that have been selected in advance to the target culture medium, will be described. In this case, the state inference system 1a performs mass spectrometry by using the measurement unit 2 for the three types of additives, obtains mass spectra, and acquires additive measurement vectors in which the amount of information of the mass spectra has been reduced, through the state inference unit 10a.

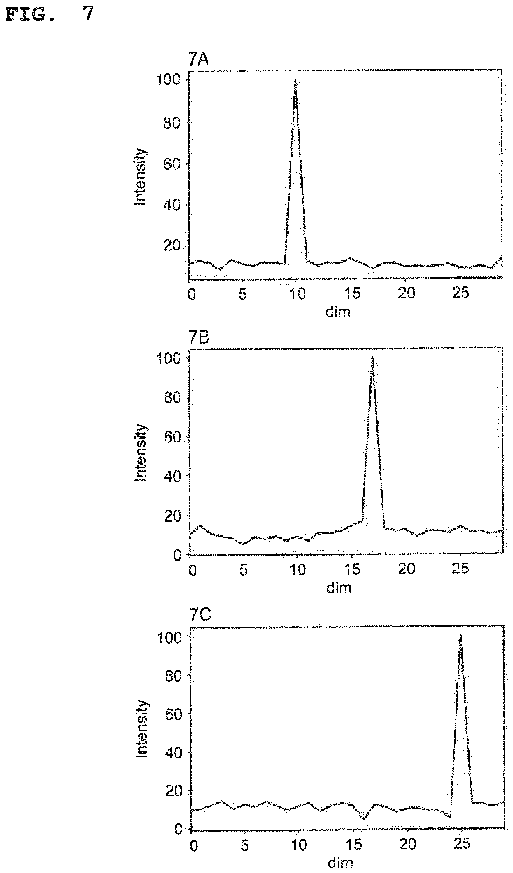

FIG. 7 is a graph showing examples of additive measurement vectors related to three types of additives, and 7A in FIG. 7 shows the additive measurement vector of a first additive, 7B in FIG. 7 shows the additive measurement vector of a second additive, while 7C in FIG. 7 shows the additive measurement vector of a third additive. The three types of the additive measurement vectors obtained in the state inference unit 10a are stored in the database 4.

The measurement target optimization inference unit 14 calculates the relevance (degree of similarity) between the additive measurement vectors, which are mass spectrometry data related to a plurality of types of additives, and the principal component vector defined in the state inference space for the feature amount extraction model. The measurement target optimization inference unit 14 infers an additive with the additive measurement vector having the greatest relevance with the base of the feature amount extraction model among the three types of additives, as an optimal additive to be added to the target culture medium. In this way, by adding an additive with the additive measurement vector having the greatest relevance with the base of the feature amount extraction model to the target culture medium before use in culturing, the culture result can be improved.

Here, the additive with the additive measurement vector having the greatest relevance with the base of the feature amount extraction model is an additive having the additive measurement vector having the highest degree of similarity with the principal component vector defined in the state inference space in the feature amount extraction model. According to the present embodiment, the degree of similarity between the principal component vector of the feature amount extraction model and the additive measurement vector is determined by calculating the inner product between the principal component vector of the feature amount extraction model and the additive measurement vector.

In a case where there are a plurality of principal component vectors in the feature amount extraction model, for example, the principal component vector having the largest eigenvalue may be selected, and the inner product between the selected principal component vector and the additive measurement vector may be calculated. In addition, the inner product with the additive measurement vector may be calculated for each principal component vector, and a total sum of adding the calculated inner products may be determined. In this case, the additive having the largest value of total sum among the total sums determined for each of the additive measurement vectors is selected as a correction candidate additive.

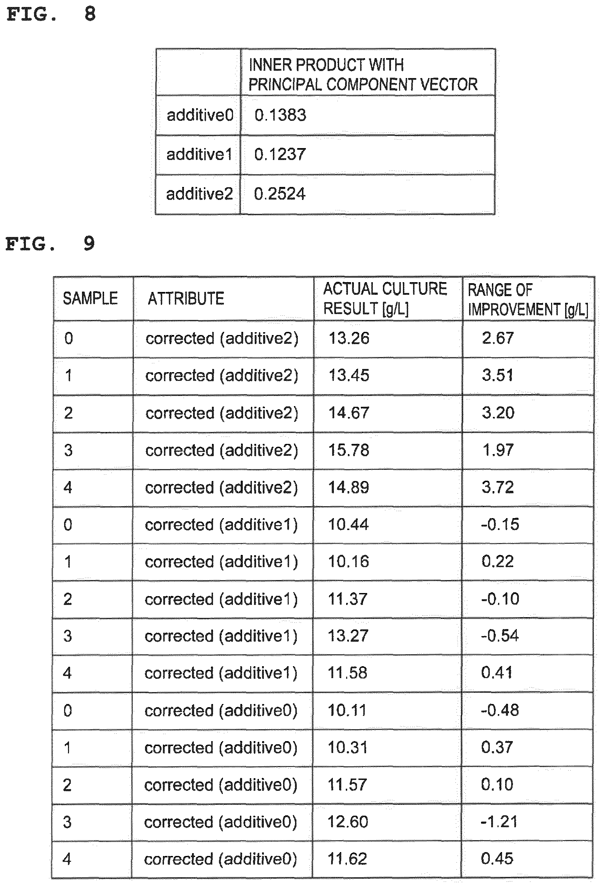

FIG. 8 is a table summarizing examples of the inner products between the principal component vector of the feature amount extraction model and the three types of additive measurement vectors. In FIG. 8, the first additive is indicated as "additive0", the second additive is indicated as "additive1", and the third additive is indicated as "additive2". Here, the third additive "additive2" has the largest inner product with the principal component vector of the feature amount extraction model and has the additive measurement vector having the greatest relevance with the principal component vector of the feature amount extraction model.

In this way, the measurement target optimization inference unit 14 calculates the inner products between the principal component vector of the feature amount extraction model and the additive measurement vectors as the degree of similarity between the principal component vector of the feature amount extraction model and the additive measurement vectors, and selects the additive whose inner product is the largest. The measurement target optimization inference unit 14 regards the selected additive as a correction candidate additive and outputs the information on the additive, such as the name of the additive, to the communication unit 5. The communication unit 5 communicates the information on the additive received from the measurement target optimization inference unit 14 to the operator or the like by displaying the content or the like.

Here, FIG. 9 shows examples of the results of the yield of Escherichia coli per unit volume of the culture medium included in each target culture medium after use in culturing, which are obtained by adding three types of additives having the additive measurement vectors shown in FIG. 7 to the five types of target culture media (samples 0 to 4) shown in FIG. 5, and actually culturing Escherichia coli by using each of the target culture media, in which the composition has been corrected with the additives.

The attribute in FIG. 9 indicates that an additive has been added to each of the target culture media prepared as samples to correct the components, and for example, the "corrected(additive2)" indicates that the third additive has been added to the sample, the "corrected(additive1)" indicates that the second additive has been added to the sample, while the "corrected(additiveO)" indicates that the first additive has been added to the sample.

In this example, by adding the third additive (additive2) having the additive measurement vector with the greatest relevance with the principal component vector of the feature amount extraction model to the target culture medium, the yield of Escherichia coli per unit volume of the culture medium included in each target culture medium after use in culturing is increased, and the range of improvement is larger than those of the other additives.

As described above, in the state inference system 1a, even when it is inferred by the state inference unit 10a that a desired culture state cannot be obtained in the target culture medium, a correction technique capable of improving the culture state by adding an optimal additive to the target culture medium may be suggested to the operator or the like. As a result, the operator may execute a culturing process in a target culture medium with enhanced performance, by adding the optimal additive inferred by the measurement target optimization inference unit 14 to the target culture medium, and can obtain a desired culture state.

In the above-mentioned embodiment, a case in which the degree of similarity between the principal component vector defined in the state inference space in the feature amount extraction model and each of a plurality of types of additive measurement vectors is calculated, and an additive with the additive measurement vector having a high degree of similarity with the principal component vector is specified by the measurement target optimization inference unit 14, has been described; however, the invention is not limited to this. For example, when information on one additive or a plurality of additives is associated in advance with the inference result for the culture state that will be obtained by the state inference unit 10a, and an inference result for the culture state is obtained from the mass spectrometry data of the target culture medium on the basis of the feature amount extraction model, the one additive or the plurality of additives that are associated in advance with the inference result may be inferred as the optimal additive to be added to the target culture medium.

### (1-5) Action and Effects

With regard to the above-described configuration, the state inference system 1a is provided with: the measurement unit 2 that measures mass spectrometry data of a plurality of culture media and at least one target culture medium as characteristic values (input data); and the state inference unit 10a that infers the culture state of a culture from the characteristic values of the target culture medium on the basis of a feature amount extraction model that characterizes the distribution of the characteristic values (input data) of the plurality of culture media. In this way, since the state inference system 1a can infer in advance whether a desired culture state can be obtained in bioproduction as well as research and development using a target culture medium, the time required for making a proper decision on the target culture medium can be shortened. As a result, the efficiency of bioproduction or research and development using a culture medium can be improved.

### (1-6) Unsupervised Generation of Feature Amount Extraction Model

In the above-described embodiment, as a feature amount extraction model that characterizes the distribution of characteristic values (mass spectrometry data) of a plurality of biological samples, a case in which a feature amount extraction model is obtained by extracting a highly relevant multi-dimensional (for example, 30-dimensional) principal component vector (supervised base) for the purpose of obtaining a satisfactory culture state with a high yield of a culture per unit volume of the culture medium, has been described.

Here, for example, the state inference system 1a that performs principal component analysis (PCA) processing by using mass spectrometry data of a plurality of culture media and extracts a multi-dimensional principal component vector (unsupervised base), which characterizes the distribution of the mass spectrometry data, to obtain a feature amount extraction model, will be described. With regard to measurement target optimization inference processing that is performed in the measurement target optimization inference unit 14 by utilizing the state inference result obtained on the basis of the feature amount extraction model, since the processing is the same as that in the above-mentioned embodiment, any further description will not be repeated here.

In this case, mass spectrometry of a plurality of culture media is performed by the measurement unit 2, for example, multivariate analysis is performed by using the obtained plurality of mass spectrometry data, and a feature amount extraction model that can clearly detect differences between the culture media is established. Specifically, when the state inference unit 10a receives mass spectra obtained by mass spectrometry from the measurement unit 2 or the database 4, the state inference unit 10a performs standardization and coarse graining on the mass spectra and generates a measurement vector in which the amount of information on the signal intensity has been reduced, as the mass spectrometry data. As the state inference unit 10a performs PCA processing on the measurement vectors of a plurality of culture media, the state inference unit 10a extracts, as a feature amount, a principal component vector (unsupervised base) that characterizes the distribution of the mass spectrometry data to generate a feature amount extraction model.

In the state inference system 1a, when mass spectrometry data of a target culture medium whose components are unknown are obtained thereafter, it can be inferred, on the basis of the mass spectrometry data of this target culture medium and the feature amount extraction model, to which culture medium the target culture medium whose components are unknown has high similarity in comparison with a past culture medium.

In this way, even for the feature amount extraction model generated by the PCA processing, the determination unit 12 can set a threshold value for abnormality determination in the state inference space of the feature amount extraction model on the basis of a certain rule for abnormality determination. In this case, the threshold value is simply defined as a quantity related to the distribution obtained when the mass spectrometry data of each of a plurality of culture media is projected onto the state inference space in which the principal component vector is defined.

For example, when the average of a point cloud obtained when the mass spectrometry data of each culture medium is projected onto the state inference space in which a plurality of principal component vectors extracted based on the mass spectrometry data is designated as µ, the threshold value for an abnormality can be given as a quantity related to the Mahalanobis distance from this µ.

Specifically, for example, in a case where a point cloud obtained by projecting mass spectrometry data is classified into a normal group and an abnormal group, and the mass spectrometry data of the normal group is projected onto a certain one-dimensional space to approximate the distribution to a normal distribution, when the average value of the distribution is designated as µ, and the absolute value of the standard deviation of the normal distribution is designated as σ, µ ± 3σ can be set as the threshold value. That is, the threshold can be set such that when the mass spectrometry data of a certain culture medium is projected onto the above-described one-dimensional space, and the coordinates are separated from *µ* by 3σ or more, the culture medium is decided to be abnormal.

The determination unit 12 performs discrimination of the presence or absence of an abnormality by using the set threshold value and a coordinate x obtained when the mass spectrometry data of the target culture medium obtained from the state inference unit 10a are projected onto the state inference space. For instance, in a case where the distance between the coordinate x and the average value µ of the distribution of the normal group is separated by 3 or more in terms of the Mahalanobis distance of the distribution of the normal group, it is determined that there is an abnormality. Otherwise, it is determined that there is no abnormality.

The degree of abnormality of the target culture medium may be determined by setting the above-described threshold value to be divided at a plurality of stages. For instance, in a case where the distance between the coordinate x and the average value µ of the distribution of the normal group is 2 or more and less than 3 in terms of the Mahalanobis distance of the distribution of the normal group, the degree of abnormality may be determined to be low, and in a case where the distance is 3 or more, the degree of abnormality may be determined to be high. The determination unit 12 outputs a determination result regarding the presence or absence of an abnormality or the degree of abnormality of the target culture medium.

### (1-7) Other Embodiments Regarding Measurement Time for Characteristics of Biological Sample

### (1-7-1) Case of Measuring Characteristics of Culture Medium During Culturing Process After Start of Culturing Process

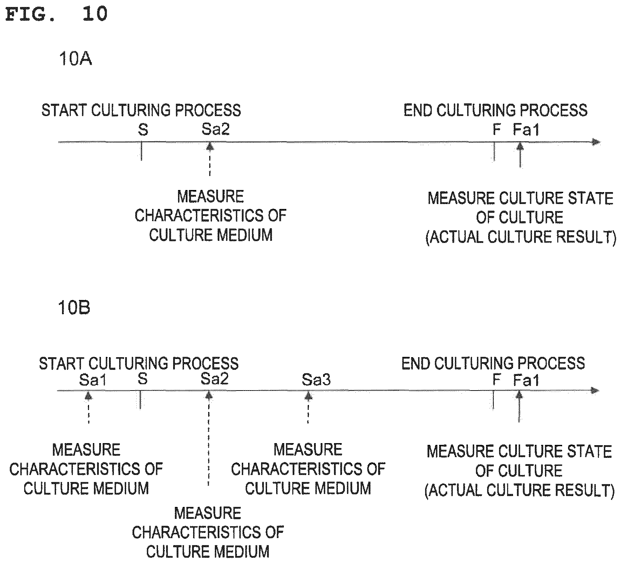

In the above-described embodiment, a case in which mass spectrometry of a culture medium is performed by the measurement unit 2 before a culturing process is started, to obtain mass spectrometry data has been described; however, the invention is not limited to this. In the above-described embodiment, for example, as shown in 10A in FIG. 10, after the culturing process is started, mass spectrometry on the culture medium in the middle of being used in the culturing process may be performed by the measurement unit 2 to obtain mass spectrometry data.

In this case, in the state inference system 1a, as shown in 10A in FIG. 10, mass spectrometry of a plurality of culture media is performed by the measurement unit 2, and mass spectrometry data are acquired, at a certain time point Sa2 (S in FIG. 10 represents the start timing of the culturing process as in FIG. 2) in the middle of performing the culturing process. In this way, in the state inference system 1a, mass spectrometry data at a certain time point Sa2 after a predetermined period has elapsed from the start of culturing continues to accumulate, in regard to a plurality of culture media in which a culture is currently being cultured by a culturing process.

In a predetermined culturing process, a culture is cultured until the end timing of the originally planned culture period, when culturing of the culture in each culture medium is ended, the culture after the end of culturing included in the culture medium is collected, and the yield of the culture that has been cultured by the culturing process is measured (Fa1). In this way, the state of each culture after culturing is measured, and an actual culture result is obtained for each culture medium.

In the state inference system 1a, when the yield (actual culture result) of the culture obtained from each culture medium is inputted by the operator or the like, the result of the culture state is stored in the database 4. At this time, in the database 4, the mass spectrometry data of each culture medium measured at a certain time point Sa2 during the culturing process, is stored in association with the final culture result obtained by the culture medium.

In the state inference unit 10a according to this embodiment, the mass spectrometry data of each culture medium measured at a certain time point Sa2 during the culturing process and the final culture state obtained by using the culture medium are read out from the database 4 as sample data to generate a feature amount extraction model.

This feature amount extraction model utilizes mass spectrometry data of a target culture medium at a certain time point Sa2 (for example, 10^{th} day from the start of culturing) after culturing of a culture is started by a predetermined culturing process, as the input, and an inference value of the future culture state finally obtainable when it is assumed that culturing of the culture is continued as it is by using the target culture medium, as the output.

As the feature amount extraction model used herein, similarly to the above-mentioned embodiment, for example, various feature amount extraction models such as PCA, PLS, polynomial regression, Gaussian process regression, and random forest regression; and a feature amount extraction model generated by machine learning of characteristic values (mass spectrometry data) of the culture medium in the middle of culturing and the culture result thereof, can be applied. When a feature amount extraction model is generated by supervised learning, it is desirable to allow the feature amount extraction model to learn the correspondence relation between the mass spectrometry data of a plurality of culture media and the culture results obtained when these culture media are used, and characterize the distribution of the mass spectrometry data from the culture results.

For example, in the case of supervised learning, in the state inference unit 10a, the mass spectrometry data of the culture medium in the middle of culturing of a culture by a culturing process are used as an explanatory variable, and the actual final culture state (yield of the culture included in the culture medium at the time of ending culturing) obtained by this culturing process is used as an objective variable, to generate in advance a feature amount extraction model that shows characteristic relevance such as a correlation or an inverse correlation between the mass spectrometry data and the culture state.

In this case as well, regarding the feature amount extraction model, unsupervised learning may be applied in addition to supervised learning, as described above. When a feature amount extraction model is generated by unsupervised learning, it is desirable to use only the mass spectrometry data of a plurality of culture media and allow learning that characterizes the distribution of a plurality of these mass spectrometry data.

As a result, in the state inference system 1a, thereafter, in the middle of performing a culturing process by using a target culture medium whose culture result is unknown, it is possible to infer the future culture result that will be finally obtained by the target culture medium, by utilizing the feature amount extraction model, in the middle of culturing before the culturing process is ended.

For example, in a series of culturing processes for inducing differentiation of cell culture and obtaining intended cells, a long time is required to obtain the intended cells. Particularly, a series of culturing processes for inducing differentiation of undifferentiated human stem cells and obtaining intended cells usually takes a time of about three months or longer. In such a culturing process, for example, when it is attempted to obtain an optimum culture result (that is, intended cells) by sequentially optimizing various parameters such as the components of the culture medium and the culture temperature, a method of waiting for the end of the culturing process, analyzing the actual culture result obtained, and determining the next parameters (components of the culture medium to be used, culture temperature, and the like) each time, may be conceived.

However, in this method, for example, in a case where one cycle of the culturing process from the start of culturing to the end of culturing is about 3 months, only about 4 cycles can be carried out at the maximum in one year, and when each parameter of the culturing process gets to be adjusted by trial and error in 4 cycles, the time efficiency is poor.

In contrast, in the state inference system 1a according to this embodiment, since an inference result for the culture state that will be finally obtained by the target culture medium is obtained in the middle of the culturing process on the basis of the feature amount extraction model, by suggesting this inference result to the operator or the like in the middle of culturing, various parameters (components of the culture medium to be used, culture temperature, and the like) of the culturing process can be changed as necessary based on the obtained inference result, without waiting for the end of the culturing process, and a new culturing process can be carried out. In this way, a new culturing process in which the parameters of the culturing process have been adjusted by trial and error so as to obtain a desired culture result, can be started in the middle of the culturing process that is currently being performed, without waiting for the end of the culturing process, and parameter optimization can be carried out in a time-efficient manner.

The above-mentioned "(1-2) State Inference Unit" uses the mass spectrometry data of the culture medium before the start of the culturing process; however, the (1-2) State Inference Unit can also be applied to this embodiment by replacing the mass spectrometry data with the mass spectrometry data of the culture medium obtained in the middle of the culturing process. In addition, similarly, the "(1-3) Determination Unit" and the "(1-4) Measurement Target Optimization Inference Unit" can also be applied by replacing the mass spectrometry data of the culture medium before the start of the culturing process with the mass spectrometry data of the culture medium obtained in the middle of the culturing process.

For example, the determination unit 12 determines the state of the target culture medium on the basis of the state inference result obtained in the state inference unit 10a from the mass spectrometry data of the culture medium during the culturing process. The measurement target optimization inference unit 14 infers an additive to be added to the target culture medium (measurement target optimization inference processing) on the basis of the state inference result obtained in the state inference unit 10a from the mass spectrometry data of the culture medium during the culturing process, in order to have a target culture medium in which a desired culture state is obtained.

### (1-7-2) Case of Measuring Characteristics of Culture Medium Multiple Times During Culturing Process

In the above-described embodiment, a case in which mass spectrometry of the culture medium is performed by the measurement unit 2 either before the culturing process is started or during the culturing process, and mass spectrometry data are obtained, has been described; however, the invention is not limited to this. In the above-described embodiment, for example, as shown in 10B in FIG. 10, mass spectrometry may be performed by the measurement unit 2 on each culture medium at a certain time point Sa1 before the culturing process is started and at certain time points Sa2 and Sa3 during the culturing process, and mass spectrometry data may be obtained a plurality of times. In addition, it is also acceptable that mass spectrometry of the culture medium by the measurement unit 2 is not performed before the culturing process is started, mass spectrometry is performed by the measurement unit 2 on each culture medium only at certain time points Sa2 and Sa3 during the culturing process, and mass spectrometry data are obtained a plurality of times. That is, mass spectrometry data of the culture medium may be measured a plurality of times at certain intervals along the timeline from the start of the culturing process, such as the mass spectrometry data of the culture medium on the 10^{th} day from the start of the culturing process and the mass spectrometry data of the culture medium on the 20^{th} day from the start of the culturing process.

In this case, in the state inference system 1a, as shown in 10B in FIG. 10, mass spectrometry of a plurality of culture media is performed by the measurement unit 2 at each of a certain time point Sa1 before the culturing process is started and certain time points Sa2 and Sa3 in the middle of carrying out the culturing process, and mass spectrometry data are acquired. In addition, in the state inference system 1a, after the end of the culturing process, the respective culture state of the culture (yield or quality of culture) after being actually cultured by using each culture medium is measured (Fa1).

In the state inference unit 10a, a feature amount extraction model is generated each separately on the basis of the mass spectrometry data of the culture medium at a certain time point Sa1 before the start of the culturing process and each of the mass spectrometry data of the culture medium at certain time points Sa2 and Sa3 after the start of the culturing process obtained in this way. That is, for example, in the case of supervised learning, the state inference unit 10a uses the mass spectrometry data of the culture medium before the start of the culturing process as an explanatory variable, and uses the culture state of the culture included in the culture medium after the end of the culturing process (after use in culturing) as an objective variable, to generate a feature amount extraction model that shows the relevance between the mass spectrometry data and the culture state. Thereafter, when mass spectrometry data measured before the start of the culturing process on a target culture medium whose components are unknown are obtained, from this mass spectrometry data, the state inference unit 10a infers the culture state of the culture (yield or quality of the culture) that will be obtained in the future when the target culture medium is used in the culturing process, on the basis of the feature amount extraction model.

In the same manner, the state inference unit 10a generates a feature amount extraction model showing the relevance between each mass spectrometry data of the culture medium at a certain time point Sa2 after the start of the culturing process and the culture state of the culture included in the culture medium after the end of the culturing process (after use in culturing), and similarly generates a feature amount extraction model showing the relevance between each mass spectrometry data of the culture medium at a certain time point Sa3 after the start of the culturing process and the culture state of the culture included in the culture medium after the end of the culturing process (after use in culturing). For a target culture medium during use in culturing, whose components are unknown, the state inference unit 10a infers the culture state of the culture (yield or quality of the culture) that will be obtained in the future when the target culture medium is used in the culturing process, from each of the mass spectrometry data measured at certain time points (Sa2, Sa3) on the basis of each of the feature amount extraction models at these certain time points Sa2 and Sa3.

In this way, mass spectrometry can be performed by the measurement unit 2 on the target culture medium at each of a certain time point Sa1 before the culturing process is started and certain time points Sa2 and Sa3 during the culturing process, and while sequentially inferring the culture state of the culture after the end of the culturing process from the mass spectrometry data of the target culture medium that changes over time, on the basis of the feature amount extraction models corresponding to the certain time points Sa1, Sa2, and Sa3, the culturing process can be executed.

Here, a case in which each feature amount extraction model is generated by supervised learning has been described; however, the invention is not limited to this, and each feature amount extraction model may also be generated by unsupervised learning according to the above-described section "(1-6) Unsupervised Generation of Feature Amount Extraction Model".

### (1-8) Other Embodiments of Inferring Culture State of Culture in Middle of Culturing Before End of Culturing Process

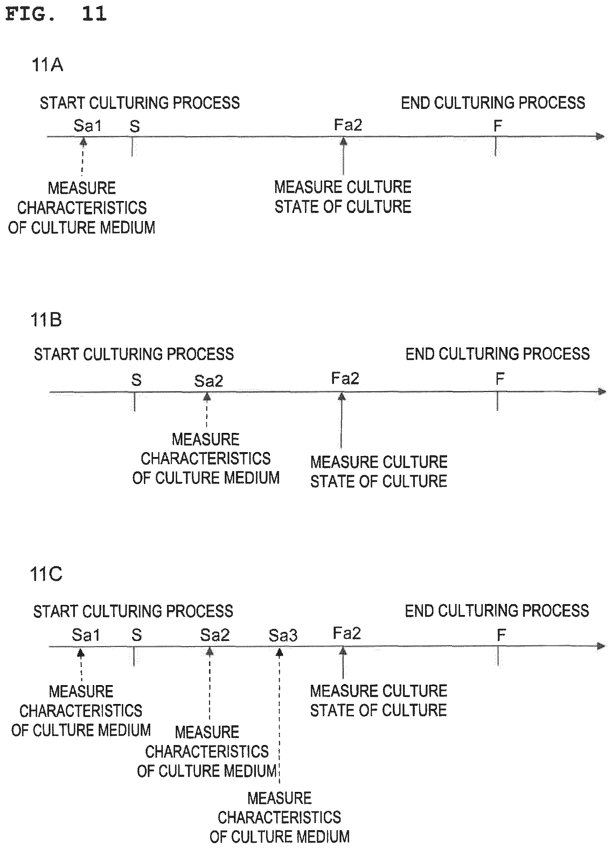

In the above-described embodiment, a case of inferring the culture state of the culture included in the culture medium (for example, yield of the culture per unit volume of the culture medium included in the target culture medium) after the culturing process is ended (after the end timing F) has been described; however, the invention is not limited to this. In the above-described embodiment, for example, as shown in 11A in FIG. 11, the culture state of the culture at a certain time point Fa2 in the middle of culturing before the culturing process is ended, may be inferred.

In this case, in the state inference system 1a, as shown in 11A in FIG. 11, mass spectrometry of a plurality of culture media is performed by the measurement unit 2 at a certain time point Sa1 before the culturing process is started, and the mass spectrometry data are acquired. In addition, in the state inference system 1a, the culture state of the culture (yield or quality of the culture) in the middle of actual culturing by each culture medium is measured at a certain time point Fa2 before the culturing process is ended.

In the state inference unit 10a, a feature amount extraction model is generated by supervised learning on the basis of the mass spectrometry data of the culture medium before the start of the culturing process and the culture state of the culture in the middle of culturing, obtained in this way. That is, the state inference unit 10a uses the mass spectrometry data of the culture medium before the start of the culturing process as an explanatory variable and uses the culture state of the culture included in the culture medium at a certain time point Fa2 before the end of the culturing process as an objective variable, to generate a feature amount extraction model showing the relevance between the mass spectrometry data and the culture state. Thereafter, when the mass spectrometry data measured before the start of the culturing process are obtained on a target culture medium whose components are unknown, the state inference unit 10a can infer the culture state of the culture in the middle of culturing (yield or quality of the culture) that will be obtained in the future when the target culture medium is used for the culturing process from the mass spectrometry data on the basis of the feature amount extraction model.

The above-described "(1-2) State Inference Unit" uses the measurement result of the culture state of the culture after the end of the culturing process; however, the same can also be applied to this "(1-8) Other Embodiments of Inferring Culture State of Culture in Middle of Culturing Before End of Culturing Process" by replacing the measurement result of the culture state with the measurement result of the culture state of the culture in the middle of culturing obtained during the culturing process. In addition, similarly, the "(1-3) Determination Unit" and "(1-4) Measurement Target Optimization Inference Unit" can also be applied by replacing the measurement result of the culture state of the culture after the end of the culturing process with the measurement result of the culture state of the culture obtained in the middle of the culturing process.

Here, as shown in 11A in FIG. 11, a case in which mass spectrometry of a plurality of culture media is performed by the measurement unit 2 at a certain time point Sa1 before the culturing process is started, and mass spectrometry data are acquired, has been described; however, the invention is not limited to this. According to this embodiment as well, for example, as shown in 11B in FIG. 11, mass spectrometry of a plurality of culture media may be performed by the measurement unit 2 at a certain time point Sa2 in the middle of performing the culturing process, and mass spectrometry data may be acquired.

In this case, the state inference unit 10a uses the mass spectrometry data of the culture medium at a certain time point Sa2 in the middle of performing the culturing process as an explanatory variable, and uses the culture state of the culture included in the culture medium at a certain time point Fa2 before the end of the culturing process as an objective variable, to generate a feature amount extraction model that characterizes the relevance between the mass spectrometry data and the culture state. Thereafter, when mass spectrometry data measured at a certain time point Sa2 in the middle of performing the culturing process are obtained on a target culture medium whose components are unknown, the state inference unit 10a can infer the culture state of the culture at a certain time point Fa2 in the middle of culturing, which will be obtained in the future when the target culture medium is used in the culturing process, from the mass spectrometry data on the basis of the feature amount extraction model.

Even in this embodiment, as shown in 11C in FIG. 11, mass spectrometry may be performed by the measurement unit 2 on the culture media at each of a certain time point Sa1 before the culturing process is started and certain time points Sa2 and Sa3 during the culturing process, and mass spectrometry data may be obtained a plurality of times. In addition, it is also acceptable that mass spectrometry of the culture medium is not performed by the measurement unit 2 before the culturing process is started, mass spectrometry is performed by the measurement unit 2 on the culture medium only at each of the time points Sa2 and Sa3 during the culturing process, and mass spectrometry data are obtained a plurality of times.

The state inference unit 10a uses the mass spectrometry data of the culture medium before the start of the culturing process as an explanatory variable, and uses the culture state of the culture included in the culture medium at a certain time point Fa2 before the culturing process is ended, as an objective variable, to generate a feature amount extraction model that characterizes the relevance between the mass spectrometry data and the culture state. Thereafter, when mass spectrometry data measured before the start of the culturing process on a target culture medium whose components are unknown are obtained, from this mass spectrometry data, the state inference unit 10a can infer the culture state of the culture (yield or quality of the culture) at a certain time point Fa2 in the middle of culturing, which will be obtained in the future when the target culture medium is used in the culturing process, on the basis of the feature amount extraction model.

The state inference unit 10a generates a feature amount inference model that characterizes the relevance between each mass spectrometry data of the culture medium at a certain time point Sa2 after the start of the culturing process and the culture state of the culture included in the culture medium at a certain time point Fa2 before the culturing process is ended, and similarly generates a feature amount extraction model that characterizes the relevance between each mass spectrometry data of the culture medium at a certain time point Sa3 after the start of the culturing process and the culture state of the culture included in the culture medium at a certain time point Fa2 before the culturing process is ended. Thereafter, when mass spectrometry data measured at each of certain time points (Sa2, Sa3) are obtained for a target culture medium during use in culturing, whose components are unknown, the state inference unit 10a can infer, from the mass spectrometry data, each of the culture states of the culture (yield of quality of the culture) at a certain time point Fa2 in the middle of culturing, which will be obtained in the future when the target culture medium is used in the culturing process, on the basis of the feature amount extraction models at the corresponding certain time points Sa2 and Sa3.

### (1-9) Optimization Inference Processing of Culture Parameters

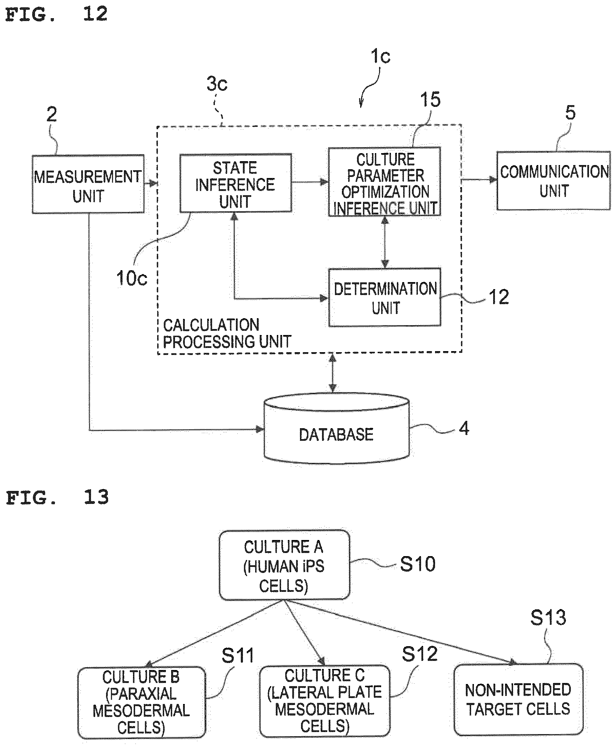

Next, a state inference system according to another embodiment, which performs optimization inference processing of culture parameters, will be described. FIG. 12 is a block diagram showing the configuration of a state inference system 1c according to another embodiment. The state inference system 1c is different from the above-described state inference system 1a in FIG. 1 in the configuration of a calculation processing unit 3c, and here, the following description will be focused on this calculation processing unit 3c, while the description on the measurement unit 2, the database 4, and the communication unit 5 will not be repeated because it is redundant.

Here, for example, when cells that serve as a culture are cultured by using a culture medium, there are circumstances in which the culturing conditions for causing the cells to reach a certain specific culture state are examined. For example, with regard to continuous culture of CHO cells, it is necessary to examine the optimal culturing conditions for causing the CHO cells to proliferate to a certain number after introducing the CHO cells into an initial culture medium. At this time, the parameters related to the culturing conditions of the culturing process (culture parameters) are subjected to trial and error so as to obtain a desired culture state, and when a new culturing process is started from the beginning each time, it requires time to specify the optimal culturing conditions. Therefore, it is desirable to carry out optimization of the culture parameters in a time-efficient manner.

With regard to the parameters (culture parameters) related to culturing conditions such as the culture temperature and culture time of the culturing process, pH of the culture medium, and the stirring speed of the culture medium, the state inference system 1c in FIG. 12 according to the present embodiment can infer the culture parameters with which a desired culture state is obtained, at the calculation processing unit 3c, and can suggest the inference result to the operator or the like through the communication unit 5. As a result, the culturing process can be carried out by using the optimal culture parameters with which a desired culture state is obtained.

The calculation processing unit 3c according to the present embodiment has a state inference unit 10c, the determination unit 12, and a culture parameter optimization inference unit 15. In a case where culture parameter optimization inference processing is performed in the calculation processing unit 3c, it is necessary to generate a feature amount extraction model for culture parameter inference in the state inference unit 10c, and this will be described below.

### (1-9-1) Generation of Feature Amount Extraction Model for Culture Parameter Inference

For example, a set of culture parameters that serve as references, such as the culture temperature and culture time at the time of culturing the culture, pH of the culture medium, and the stirring speed of the culture medium, are determined. In addition, a plurality of correction candidate values Δa of the culture parameters are determined from these culture parameters that serve as references. Specifically, the correction candidate value Δa of the culture temperature is, for example, +2°C or - 1°C; the correction candidate value Δa of the culture time is, for example, +30 minutes or -30 minutes; the correction candidate value Δa of the pH of the culture medium is +0.1 or -0.2; and the correction candidate value Δa of the stirring speed of the culture medium is, for example, +1 s⁻¹ or -1 s⁻¹.

Here, as an example, a case in which CHO cells are added as a culture to 20 culture media, and culturing of the culture (CHO cells) is started with a certain culture parameter a, will be described. At a time point where 3 hours have elapsed from the start of culturing, the supernatants are taken, mass spectrometry is performed on each of the culture media by the measurement unit 2, which is a mass spectrometer, and mass spectra are obtained. Then, at the time point where 3 hours have elapsed from the start of culturing, the cultures are subjected to subculture and dispensation.

Here, the culture fluids of the 20 culture media are each divided into four parts, and each part is transferred to a separate culture container. For each of the culture containers, four different culture parameters (a+Δa0 (here, Δa0 = 0)), (a+Δa1), (a+Δa2), and (a+Δa3) are respectively set, and culturing in each culture container is continued with these culture parameters (a + Δa0), (a+Δa1), (a+Δa2), and (a+Δa3). Here, each of Δa1, Δa2, and Δa3 represents a different correction candidate value of a culture parameter, and for example, when the culture temperature is adopted as the culture parameter, each of Δa1, Δa2, and Δa3 indicates a temperature rise or a temperature drop from the reference culture temperature.

The actual yield of the culture per unit volume of the culture medium (culture state) obtainable when performing the culturing process with each of the culture parameters (a+Δa0), (a+Δa1), (a+Δa2), and (a+Δa3) is obtained, and this is stored in the database 4 as an actual state result. As a result, 20 × 4 = 80 types of actual state results for learning of culturing in all of the 20 culture media with the four culture parameters (a+Δa0), (a+Δa1), (a+Δa2), and (a+Δa3) for each culture medium, are obtained.

The mass spectrometry data of the culture media, the reference culture parameter a, the correction candidate values Δa0, Δa1, Δa2, and Δa3 of the culture parameter, and the actual state results indicating the culture state of the culture when the culture is actually cultured with the culture parameters (a+Δa0), (a+Δa1), (a+Δa2), and (a+Δa3), are associated correspondingly, and each of these combinations is stored in the database 4 as a data set.

Next, the state inference unit 10c reads out these data sets stored in the database 4 and uses the mass spectrometry data, the culture parameter a before correction, which serves as a reference, and the correction candidate values of the culture parameter a, namely, Δa0, Δa1, Δa2, and Δa3, as the input data, to generate a feature amount extraction model for culture parameter inference, which characterizes the distribution of these input data. Specifically, a feature amount extraction model for culture parameter inference is generated, which infers, for a culture obtained 3 hours after the start of culturing, what correction is applied to the reference culture parameter for obtaining a desired culture state.

Here, a feature amount extraction model for culture parameter inference is generated by using the mass spectrometry data, a reference culture parameter a before correction, and the correction candidate values of the culture parameter a (Δa0, Δa1, Δa2, and Δa3) as explanatory variables; however, the invention is not limited to this, and a feature amount extraction model for culture parameter inference may also be generated by using the mass spectrometry data and the culture parameters (a+Δa0), (a+Δa1), (a+Δa2), and (a+Δa3) as explanatory variables. In addition, the feature amount extraction model for culture parameter inference is the same in terms that, for example, various feature amount extraction models such as PCA, PLS, polynomial regression, Gaussian process regression, conventional neural network, and Bayesian neural network can be applied, as described in the above-described section "(1-1) Configuration of State Inference System".

For example, here, the state inference unit 10c generates a feature amount extraction model for culture parameter inference, which uses the mass spectrometry data of the culture media obtained by the measurement unit 2, a reference culture parameter a before correction, and correction candidate values Δa0, Δa1, Δa1, and Δa1 as the input (explanatory variable), and the culture state of the culture (yield in this case) included in the culture medium after use in culturing as output (objective variable), through a neural network or the like.

In this way, the state inference system 1c generates a feature amount extraction model for culture parameter inference, in which for input data such as the mass spectrometry data of the culture media and culture parameters (a+Δa0), (a+Δa1), (a+Δa2), and (a+Δa3) that are set in a culturing process, the distribution of the input data is characterized from the culture state of a culture cultured in the culturing process.

### (1-9-2) Application of Feature Amount Extraction Model for Culture Parameter Inference

When mass spectrometry data of a target culture medium whose components are unknown are obtained through the measurement unit 2, the state inference system 1c randomly selects a correction candidate value Δa of the culture parameter within a preliminarily set range through a culture parameter optimization inference unit 15, and outputs a plurality of correction candidate values Δa to the state inference unit 10c. Here, a case in which the correction candidate values Δa of the culture parameter have been randomly generated at the culture parameter optimization inference unit 15 has been described; however, the invention is not limited to this, and for example, a plurality of correction candidate values Δa that has been stored in advance in the database 4 may be applied, or correction candidate values Δa selected by the operator or the like may be used.

The state inference unit 10c infers the culture state (yield) of a culture included in a culture medium used in culturing, by inputting mass spectrometry data of a target culture medium obtained through the measurement unit 2, a culture parameter a, and a plurality of correction candidate values Δa of the culture parameter as the input data into the above-mentioned feature amount extraction model for culture parameter inference, and performing analysis.

The state inference unit 10c changes the correction candidate values Δa and then outputs each of the obtained plurality of inference results into the culture parameter optimization inference unit 15. The culture parameter optimization inference unit 15 selects, among the plurality of inference results obtained in the state inference unit 10c, an inference result that is closest to the result of the optimal culture state that has been set in advance, and specifies the correction candidate value Δa of the culture parameter a set with the selected inference result.

The culture parameter optimization inference unit 15 may output the specified correction candidate value Δa into the communication unit 5 and suggest this correction candidate value Δa to the operator or the like through the communication unit 5. In this way, by suggesting a correction candidate value Δa obtained in the culture parameter optimization inference unit 15 to the operator or the like through the communication unit 5, the operator or the like can correct the culture parameter based on the correction candidate value Δa and can culture a culture under the optimal culturing conditions (culture parameter).

### (1-9-3) Action and Effects

With regard to the above-described configuration, the state inference system 1c uses the characteristic values of a plurality of culture media measured in the measurement unit 2 and a plurality of culture parameters as the input data, and infers the culture state of a culture in the state inference unit 10c from the characteristic values of a target culture medium and the culture parameters on the basis of a feature amount extraction model for culture parameter inference, which characterizes the distribution of those input data. In this way, in the state inference system 1c, since it can be inferred in advance whether a desired culture state can be obtained in bioproduction as well as research and development using a target culture medium, the time required for making a proper decision on the target culture medium can be shortened. As a result, the efficiency of bioproduction or research and development using a culture medium can be improved.

In addition to this, this state inference system 1c specifies a correction candidate value Δa of the culture parameter a, with which an optimal culture state is obtained, on the basis of the inference result for the culture state obtained by the state inference unit 10c. As a result, in the state inference system 1c, the culture parameter can be corrected by the operator or the like based on the obtained correction candidate value Δa of the culture parameter, and an optimal culture state can be obtained.

According to this embodiment, for example, the inference result obtained in the state inference unit 10c may be outputted to the determination unit 12, and culture parameters may be determined on the basis of the inference result in the determination unit 12. In this case, the culture parameters are determined on the basis of the inference result by the state determination processing or abnormality determination processing described in the above-described section "(1-3) Determination Unit". For example, in the case of performing state determination processing, the determination unit 12 sets identification values that enable stepwise identification between a satisfactory inference result and a poor inference result, in a state inference space in which a principal component vector is defined as a feature amount for the feature amount extraction model for culture parameter inference, prior to state determination processing. The determination unit 12 selects an identification value close to the value of a state inference vector obtained by projecting the mass spectrometry data of the target culture medium and the culture parameter onto the state inference space, and identifies an inference result from the selected identification value to determine the appropriateness of the culture parameter.

As the abnormality determination processing, the determination unit 12 may set a threshold value of the abnormality determination in the state inference space of the feature amount extraction model for culture parameter inference on the basis of a certain rule for abnormality determination. The determination unit 12 identifies an inference result from the value of a state inference vector obtained by projecting the mass spectrometry data of the target culture medium and the culture parameter onto a state inference space in which the principal component vector is defined, and from a threshold value, and determines the presence or absence of abnormality in the culture parameter. In addition, it is also acceptable that the degree of abnormality in the culture parameter may be determined by setting the above-described threshold value to be divided at a plurality of stages.

### (1-10) State Inference System Inferring Culture Time

In the above-described embodiment, the state inference systems 1a and 1c that infer the culture state of a culture on the basis of a feature amount extraction model have been each described; however, the invention is not limited to this, and the state inference system may be a state inference system that infers the culture time required until a culture that is being cultured in a culturing process reaches a preliminarily set culture state, on the basis of a feature amount extraction model.

In this case, in each of the sections "(1-1) Configuration of State Inference System", "(1-2) State Inference Unit", "(1-3) Determination Unit", "(1-4) Measurement Target Optimization Inference Unit", "(1-5) Action and Effects", "(1-6) Unsupervised Generation of Feature Amount Extraction Model", "(1-7) Other Embodiments Regarding Measurement Time for Characteristics of Culture Medium", and "(1-9) Optimization Inference Processing of Culture Parameters" as described in the above-described "(1) First Embodiment", the processing of each of these sections can be executed by changing the "culture state (yield) of the culture" that serves as an objective variable or the like by replacing it with the "culture time required until the culture reaches a preliminarily set culture state".

### (1-10-1) Regarding State Inference System Shown in FIG. 1

For example, in a case where the state inference system 1a that infers the culture time is adopted as the state inference system 1a shown in FIG. 1, in the state inference unit 10a, in the case of supervised learning, a feature amount extraction model is generated by using the mass spectrometry data of a plurality of culture media obtained by the measurement unit 2 at a certain time point as an explanatory variable, and using the culture time in which, when a culture is actually cultured using the culture media, the culture reaches a desired culture state, as an objective variable. In such a feature amount extraction model, the components of a culture medium that affect the culture time in which, when a culture is cultured by using a culture medium, the culture reaches a desired culture state (hereinafter, simply referred to as Culture time), can be analyzed.

For the feature amount extraction model, in the case of supervised learning, it is desirable to apply a regression model or the like that uses the mass spectrometry data of a target culture medium as the input, and uses the estimation result of the culture time (hereinafter, also referred to as time inference result) taken until a culture reaches a desired culture state when it is assumed that culturing of the culture using the target culture medium has been continued as it is, as the output. In addition, when a feature amount extraction model is generated by supervised learning, it is desirable to allow the feature amount extraction model to learn the correspondence relation between the mass spectrometry data of a plurality of culture media and the culture time taken for a culture to reach a desired culture state when these culture media are used, and characterize the distribution of the mass spectrometry data from the culture time.

Regarding supervised learning of other feature amount extraction models, for example, the mass spectrometry data of a plurality of culture media, each culture time obtained when these culture media are used, and a truth label indicating whether the culture time is a desired result (for example, an accuracy indicating whether the culture time is a desired culture time) are used, and when the mass spectrometry data of the culture media and the culture time are learned, this learning may be carried out by associating the mass spectrometry data and the culture time with the truth label, so that the distribution of the mass spectrometry data may be characterized.

As the feature amount extraction model, for example, various feature amount extraction models such as PCA, PLS, polynomial regression, Gaussian process regression, and Random Forest Regression can be applied. In addition, as the feature amount extraction model, for example, a machine learning model generated by machine learning of the characteristic values (mass spectrometry data) of the culture media and the result of culture time, may also be applied.

As the machine learning model, for example, a neural network (for example, a convolutional neural network (CNN) or a Bayesian neural network (BNN)) can be applied. In addition, the feature amount extraction model may involve supervised learning as described above, as well as unsupervised learning. The details in the case of generating the feature amount extraction model by unsupervised learning will be described below; however, when the feature amount extraction model is generated by unsupervised learning, it is desirable to use the mass spectrometry data of a plurality of culture media and allow learning that characterizes the distribution of a plurality of these mass spectrometry data.

The determination unit 12 determines the state of a target culture medium on the basis of the inference result (time inference result) for the culture time taken until the culture reaches a desired culture state, which is obtained by the state inference unit 10a. The determination unit 12 sets identification values that enable stepwise identification between a satisfactory culture medium and a poor culture medium, in a state inference space in which a principal component vector (feature amount) is defined in the feature amount extraction model, the mass spectrometry data of a target culture medium are projected onto the state inference space, and whether the target culture medium is in a state of being capable of achieving a desired culture time is inferred by using the identification values as a guide (state determination processing).

Here, a culture medium in which the culture time taken until the mass of a culture per unit volume of the culture medium obtained when the culture is cultured by using a culture medium becomes equal to or greater than a predetermined optimum set value (satisfactory determination value) is within any time set in advance, is referred to as "satisfactory culture medium", and a culture medium in which the culture time is longer than any time is referred to as "poor culture medium".

The determination unit 12 may set the threshold value for the abnormality determination in the state inference space for the feature amount extraction model on the basis of a certain rule for abnormality determination. In this case, setting of the threshold value may be set in the state inference space by the operator or the like in consideration of the desired culture time. For example, setting of the threshold value is achieved such that when the threshold value is not exceeded, the culture medium may be determined as a culture medium in which the culture time is short, and a satisfactory culture time is obtained, whereas when the threshold value is exceeded, the culture medium is determined as a culture medium in which the culture time is long, and a poor culture time is obtained.

The determination unit 12 determines the presence or absence of abnormality of a target culture medium from the viewpoint of the culture time, from the value of a state inference vector obtained by projecting the mass spectrometry data of the target culture medium onto a state inference space in which a principal component vector is defined, and from the threshold value. In addition, it is also acceptable that the degree of abnormality of the target culture medium is determined by setting the above-described threshold value to be divided at a plurality of stages.

When an inference result for the culture time (time inference result) of a culture is obtained in the state inference unit 10a from the mass spectrometry data of a target culture medium before the start of the culturing process on the basis of a feature amount extraction model, the measurement target optimization inference unit 14 receives this time inference result from the state inference unit 10a. The measurement target optimization inference unit 14 analyzes the time inference result obtained in the state inference unit 10a and infers an additive that can, for example, shorten the culture time of the culture when added to the target culture medium before use in culturing. The measurement target optimization inference unit 14 allows the operator or the like to correct the components of the target culture medium used in the culturing process by adding the additive that has been inferred (also referred to as inferred additive) to the target culture medium.

In this case, the measurement target optimization inference unit 14 calculates the relevance (degree of similarity) between an additive measurement vector, which is mass spectrometry data related to a plurality of types of additives, and a principal component vector defined in a state inference space for the feature amount extraction model, according to the above-described section "(1-4) Measurement Target Optimization Inference Unit". The measurement target optimization inference unit 14 infers an additive with the additive measurement vector having the greatest relevance with the base of the feature amount extraction model among the three types of additives, as an optimal additive to be added to the target culture medium. In this way, by adding an additive with the additive measurement vector having the greatest relevance with the base of the feature amount extraction model to the target culture medium before use in culturing, the culture time can be improved.

Here, the additive with the additive measurement vector having a great relevance with the base of the feature amount extraction model is an additive having the additive measurement vector having a high degree of similarity with the principal component vector defined in the state inference space in the feature amount extraction model. According to the present embodiment, the degree of similarity between the principal component vector of the feature amount extraction model and the additive measurement vector may be determined by calculating the inner product between the principal component vector of the feature amount extraction model and the additive measurement vector, and in a case where there are a plurality of principal component vectors in the feature amount extraction model, for example, a principal component vector having the highest eigenvalue may be selected, and the inner product between the selected principal component vector and the additive measurement vector may be calculated.

In the above-described embodiment, a case in which the degree of similarity between the principal component vector defined in the state inference space in the feature amount extraction model and each of a plurality of types of additive measurement vectors is calculated, and an additive with the additive measurement vector having a high degree of similarity with the principal component vector is specified by the measurement target optimization inference unit 14, has been described; however, the invention is not limited to this. For example, when information on one additive or a plurality of additives is associated in advance with the inference result for the culture time that will be obtained by the state inference unit 10a, and an inference result for the culture time is obtained from the mass spectrometry data of the target culture medium on the basis of the feature amount extraction model, the one additive or the plurality of additives that are associated in advance with the inference result may be inferred as the optimal additive to be added to the target culture medium.

### (1-10-2) Regarding State Inference System Shown in FIG. 12

The state inference system 1c shown in FIG. 12 performs optimization inference processing of culture parameters; however, even this state inference system 1c may be allowed to infer optimal culture parameters on the basis of the inference result for the culture time according to the above-described section " (1-9) Optimization Inference Processing of Culture Parameters". In this case, in the above-described section " (1-9-1) Generation of Feature Amount Extraction Model for Culture Parameter Inference", the mass spectrometry data of a culture medium, the reference culture parameter a, the correction candidate values Δa0, Δa1, Δa2, and Δa3 of the culture parameter, and the actual state results indicating the culture time taken until a culture reaches a desired culture state when the culture is actually cultured with the culture parameters (a+Δa0), (a+Δa1), (a+Δa2), and (a+Δa3), are associated correspondingly, and each of these combinations is stored in the database 4 as a data set.

The state inference unit 10c reads out these data sets stored in the database 4 and uses the mass spectrometry data, the culture parameter a before correction, which serves as a reference, and the correction candidate values of the culture parameter a, namely, Δa0, Δa1, Δa2, and Δa3, as the input data, to generate a feature amount extraction model for culture parameter inference, which characterizes the distribution of these input data. Specifically, a feature amount extraction model for culture parameter inference is generated, which infers, for a culture obtained 3 hours after the start of culturing, what correction is applied to the reference culture parameter for obtaining a desired culture state.

In the "(1-9-2) Application of Feature Amount Extraction Model for Culture Parameter Inference", the state inference unit 10c infers the culture time taken until a culture reaches a desired culture state (yield), by inputting mass spectrometry data of a target culture medium measured through the measurement unit 2, a culture parameter a, and a plurality of correction candidate values Δa of the culture parameter as the input data into the above-mentioned feature amount extraction model for culture parameter inference, and performing analysis.

The culture parameter optimization inference unit 15 selects, among a plurality of inference results obtained in the state inference unit 10c, for example, an inference result with the shortest culture time, and specifies a correction candidate values Δa for the culture parameter a set with the selected inference result. In this way, by suggesting the correction candidate values Δa obtained in the culture parameter optimization inference unit 15 to the operator or the like through the communication unit 5, the operator or the like may correct the culture parameter based on the correction candidate values Δa and can shorten the culture time.

According to this embodiment as well, for example, the time inference result obtained in the state inference unit 10c may be outputted to the determination unit 12, and culture parameters may be determined on the basis of the time inference result in the determination unit 12. In this case, the culture parameters are determined on the basis of the time inference result by the state determination processing or abnormality determination processing described in the above-described section "(1-3) Determination Unit". Meanwhile, since the state determination processing or abnormality determination processing is described in the above-described section "(1-3) Determination Unit", description thereof will not be repeated here in order to avoid redundancy of description.

### (1-11) Others

In the above-described embodiments, a case in which culture media are applied as the biological sample and the target biological sample has been described; however, the invention is not limited to this, and for example, any of a culture medium, a culture cultured by using a culture medium, a culture medium extract extracted from a culture medium, and a culture extract extracted from a culture may be applied as the biological sample and the target biological sample.

For example, in a case where cultures such as cells are used as the biological sample and the target biological sample, mass spectrometry is performed by the measurement unit 2 on a plurality of cultures and at least one target culture, which serves as a target, at a certain time point, and mass spectrometry data are measured. The state inference unit 10a infers a future culture state of the target culture from the characteristic values of the target culture on the basis of a feature amount extraction model that characterizes the distribution of mass spectrometry data, which are characteristic values of a plurality of cultures. In addition, also for the state inference unit 10c, similarly, the characteristic values of a culture, a culture medium extract, or a culture extract may be used instead of the characteristic values of the culture medium.

A culture medium extract extracted from a culture medium includes, for example, substances included in the original culture medium raw materials, as well as the metabolites (for example, antibodies, proteins, carbohydrates, amino acids, and lipids) of microorganisms or cells including target substances and byproducts, which are generated when the culture metabolizes those substances, and substances obtained when the substances included in the original culture medium raw materials are denatured (for example, Maillard reaction) over time. When culture medium extracts are used as the biological sample and the target biological sample, mass spectrometry is performed by the measurement unit 2 on a plurality of culture medium extracts and at least one target culture medium extract, which serves as a target, at a certain time point, and mass spectrometry data are measured. The state inference unit 10a infers the future culture state of the culture from the characteristic values of the target culture medium extract on the basis of a feature amount extraction model that characterizes the distribution of mass spectrometry data, which are characteristic values of a plurality of culture medium extracts.

A culture extract extracted from a culture includes, for example, substances constituting the inner part of a culture or the culture itself, and more specifically, a culture extract includes metabolites (for example, antibodies, proteins, carbohydrates, amino acids, and lipids) obtained when a culture metabolizes culture medium raw materials, and substances that are originally included in the culture medium raw materials and are taken in by a culture into the body to be accumulated therein (for example, heavy metals). In a case where culture extracts are used as the biological sample and the target biological sample, mass spectrometry is performed by the measurement unit 2 on a plurality of culture extracts and at least one target culture extract, which serves as a target, at a certain time point, and mass spectrometry data are measured. The state inference unit 10a infers the future culture state of a culture from the characteristic values of a target culture extract on the basis of a feature amount extraction model that characterizes the distribution of mass spectrometry data, which are the characteristic values of a plurality of culture extracts.

### (2) Second Embodiment

In the above-described first embodiment, a case in which, for example, a mass spectrometer that measures mass spectrometry data of a plurality of biological samples and a target biological sample is applied as the measurement unit that measures the characteristics of a plurality of biological samples and a target biological sample, has been described; however, in the second embodiment, a case of applying a microscope apparatus that measures the appearance of a plurality of biological samples and a target biological sample will be described below.

### (2-1) Overview of State Inference Unit and Determination Unit According to Second Embodiment

In this case, in the state inference system 1a shown in FIG. 1, a microscope apparatus (for example, a fluorescence microscope apparatus) including an imaging device such as a charge coupled device (CCD) camera is applied as the measurement unit 2. The measurement unit 2, which is a microscope apparatus, captures images of a culture in order to measure an appearance state of the culture in a culture medium as a characteristic of the culture and acquires image data of the culture. In the second embodiment, the biological sample is a culture, and as the culture, for example, cells used for a cell culture experiment will be described as an example.

For example, in a cell culture experiment, cells in an undifferentiated state are cultured by a predetermined culturing process using a culture medium, and cultured cells are obtained. Then, a differentiation state of the cells can be specified by staining the cultured cells by, for example, immunostaining, and observing the manifestation state of light emitted from the cells using a microscope apparatus. In this case, it is desirable to use a fluorescence microscope apparatus capable of imaging fluorescence emitted by a culture, which is a measurement target, as the microscope apparatus.

Regarding the differentiation state of actually cultured cells, the differentiated state of the cells can be specified by, for example, analyzing metabolites from the cells by using a mass spectrometer, and furthermore, the differentiation state of the cells can be inferred by measuring and analyzing the expression state of a gene in the cells after culturing by RNA sequencing (RNA-Seq).

In the state inference system 1a according to the second embodiment, image data are obtained by imaging undifferentiated target cells by using the measurement unit 2, and these image data are used as the input for a feature amount extraction model, and the future culture state (differentiation state) of the undifferentiated target cells is inferred before reaching a differentiated state, from the image data of the target cells on the basis of a feature amount extraction model. The state inference system 1a according to the second embodiment has the measurement unit 2, which is a microscope apparatus, the calculation processing unit 3a, the database 4, and the communication unit 5, and mainly, the state inference system 1a is different in terms of using "image data" of a culture acquired with the measurement unit 2 instead of the "mass spectrometry data" of a culture medium, as described in the above-described first embodiment.

In the second embodiment, for example, a plurality of undifferentiated cells are each imaged by using the measurement unit 2, and a feature amount extraction model is generated on the basis of the obtained plurality of image data. Thereafter, the state inference system 1a can capture images of undifferentiated target cells whose state is unknown with the measurement unit 2 and infer the differentiation state (culture state) obtained when the target cells are cultured in the future, on the basis of the image data obtained as a result and the above-described feature amount extraction model. In this way, a biological sample whose culture state is inferred by using a feature amount extraction model that has been generated in advance, is referred to as "target biological sample". In the second embodiment, a case in which cells (culture) are applied as an example of the biological sample will be described below, and cells whose culture state is inferred by using a feature amount extraction model that has been generated in advance, are referred to as "target culture" or "target cells".

In this case, the measurement unit 2 captures images of each of undifferentiated cells included in each of a plurality of culture media and outputs the obtained plurality of image data to the calculation processing unit 3a and the database 4. When the image data are inputted from the measurement unit 2, the database 4 stores each image data in association with the date and time when the image data were obtained. In addition, in the database 4, information on the background of the imaged cells (the background includes, for example, the type of the cells (specification information), product number, lot number, date of manufacture, date of arrival, and past usage history) is stored, and furthermore, information on parameters related to the culturing conditions (that is, culture parameters), such as the culture temperature and culture time of the culturing process, the pH of the culture medium, and the stirring speed for the culture medium, is also stored in association with the image data.

The cells imaged by the measurement unit 2 are actually cultured by using a culture medium by a predetermined culturing process. Then, the differentiation state of the actually cultured cells is checked on the basis of image data obtained by, for example, staining the cultured cells with a staining substance and imaging the cells by using a microscope apparatus.

In the state inference unit 10a according to the second embodiment, the image data of a plurality of undifferentiated cells obtained by the measurement unit 2 at a certain time point, and the differentiation state of each of the cells obtained when the cells are actually cultured by using a culture medium, that is, the result of the culture state (culture result), are used to perform, for example, multivariate analysis, and a feature amount extraction model is generated. In such a feature amount extraction model, the appearance feature of cells, which has a risk of affecting the culture result obtained when the cells are cultured by using a culture medium, can be analyzed.

For the feature amount extraction model, in the case of supervised learning, it is desirable to apply a regression model or the like, which uses image data of target cells as the input, and the inference result of the future culture state (state inference result) finally obtainable when it is assumed to continue culturing of the target cells as it is by using a culture medium, as the output. In addition, when a feature amount extraction model is generated by supervised learning, it is desirable to allow the feature amount extraction model to learn the correspondence relation between the image data of a plurality of cells and the culture state (differentiation state) of these cells, and characterize the distribution of the appearance feature of the cells in the image data from the culture state.

Regarding supervised learning of other feature amount extraction models, for example, the image data of a plurality of cells, the culture state of each cell, and a truth label indicating whether the culture state is a desired result (for example, an accuracy indicating whether the culture state is a desired culture state) are used, and when the image data of the cells and the culture state thereof are learned, this learning may be carried out by associating the image data and the culture state with the truth label, so that the distribution of the appearance feature of the cells in the image data may be characterized.

Regarding the feature amount extraction model according to the second embodiment, similarly to the first embodiment, for example, various feature amount extraction models such as principal component analysis (PCA), partial least squares (PLS), polynomial regression, Gaussian process regression, and random forest regression can be applied. As the feature amount extraction model, for example, a machine learning model generated by machine learning of the characteristic values (image data) of the cells and the culture result of the cells, may also be applied.

As the machine learning model, for example, a neural network (for example, a convolutional neural network (CNN) or a Bayesian neural network (BNN)) can be applied.

When a feature amount extraction model is generated by supervised learning, for example, it is desirable that the data indicating the differentiation state of each cell that has been actually cultured, as an index, is applied as an objective variable. In addition, the characteristic state itself of each cell in the image data (that is, the image data itself obtained by imaging the cell, without indicating the differentiation state of an actually cultured cell with an "index") obtained by staining each cell that has been actually cultured with a staining substance and imaging the cell with the measurement unit 2, which is a microscope apparatus, may also be applied as an objective variable.

The feature amount extraction model may involve supervised learning as described above, as well as unsupervised learning. When a feature amount extraction model that implements unsupervised learning is generated, it is desirable to use the image data of a plurality of cells and allow learning that characterizes the distribution of the appearance of each cell in a plurality of these image data.

In such second embodiment, in each of the sections "(1-1) Configuration of State Inference System", "(1-2) State Inference Unit", "(1-3) Determination Unit", "(1-5) Action and Effects", "(1-6) Unsupervised Generation of Feature Amount Extraction Model", "(1-7) Other Embodiments Regarding Measurement Time for Characteristics of Biological Sample", "(1-8) Other Embodiments of Inferring Culture State of Culture in Middle of Culturing Before End of Culturing Process", "(1-9) Optimization Inference Processing of Culture Parameters", "(1-10) State Inference System Inferring Culture Time", and "(1-11) Others" as described in the above-described "(1) First Embodiment", processing of each of these sections can be applied by replacing the "mass spectrometry data" (or measurement vector) acquired through the measurement unit 2 with the "image data".

In the second embodiment, as described above, since each processing can be described by replacing the "mass spectrometry data" (measurement vector) with "image data" with regard to the "(1) First Embodiment", the detailed description of each section will not be repeated because it is redundant, and only an outline will be described below.

For example, in the "(1-3) Determination Unit" described above in the second embodiment, the state of the target cells is determined by the determination unit 12 on the basis of the state inference result obtained by the state inference unit 10a. Regarding the determination processing in the determination unit 12, there are state determination processing in which whether the cultured target cells are in a suitable culture state is simply determined stepwise, and abnormality determination processing in which whether the cultured target cells are in an abnormal culture state, such as not being in a desired differentiation state.

In a case where state determination processing is performed by applying a regression model as the feature amount extraction model, the determination unit 12 sets identification values that enable stepwise identification between a satisfactory cell and a poor cell, for example, in a state inference space in which a principal component vector (supervised base) is defined, as a feature amount, for the feature amount extraction model, prior to the state determination processing. In addition, these identification values may be associated with identification marks that indicate stepwise the degree of satisfactoriness regarding the differentiation state of cells, for example, by taking the culture result obtained when the cells are cultured by using a culture medium, as a reference.

Regarding the setting of the identification values for the state inference space, similarly to the above-described first embodiment, any numerical values may be set by the operator or the like, or the principal component vector defined in the state inference space may keep being automatically divided into sections at predetermined intervals, and the identification values may be automatically set. The determination unit 12 determines the state of the target cells from the value of the state inference vector obtained by projecting the image data of the target cells onto the state inference space, and from the identification values.

The determination unit 12 may also set a threshold value for abnormality determination in the state inference space for the feature amount extraction model, on the basis of a certain rule for the abnormality determination. In this case, setting of the threshold value may be set in the state inference space by the operator or the like in consideration of a desired culture result. For example, setting of the threshold value is achieved such that when not exceeding the threshold value, a satisfactory culture result equal to or higher than a reference value is obtained, and when exceeding the threshold value, a poor culture result less than a reference value is obtained.

The determination unit 12 determines the presence or absence of an abnormality of target cells from the value of the state inference vector obtained by incorporating image data of the target cells into the feature amount extraction model and projecting the feature amount extracted from the image data onto the state inference space in which the principal component vector is defined, and from a threshold value. In addition, the degree of abnormality of the target cells may be determined by setting the above-described threshold value to be divided at a plurality of stages.

In the same manner as in the above-described first embodiment, when a determination result for the target cells obtained by state determination processing is inputted, the communication unit 5 communicates the determination result obtained by determining the state of the target cells before use in culturing on the basis of the identification values, to the operator or the like. In addition, when the presence or absence of an abnormality based on abnormality determination processing or the determination result for the degree of abnormality is inputted, the communication unit 5 communicates the presence or absence of an abnormality or the degree of abnormality to the operator or the like.

With regard to the above-described configuration, the state inference system 1a according to the second embodiment is provided with the measurement unit 2 that measures the image data of a plurality of cultures and at least one target culture as characteristic values (input data), and the state inference unit 10a that infers the culture state of the target culture from the characteristic values of the target culture on the basis of a feature amount extraction model that characterizes the distribution of the characteristic values (input data) of the plurality of cultures. In this way, in the state inference system 1a, since it can be inferred in advance whether a desired culture state can be obtained in bioproduction as well as research and development using a target culture medium, the time required for making a proper decision on the target culture medium can be shortened. As a result, the efficiency of bioproduction or research and development using a culture medium can be improved.

### (2-2) Unsupervised Generation of Feature Amount Extraction Model According to Second Embodiment

For example, the state inference system 1a in which principal component analysis (PCA) processing is performed by using image data of a plurality of cells, and a multi-dimensional principal component vector (unsupervised base) that characterizes the distribution of the appearance state of the cells in the image data is extracted to obtain a feature amount extraction model, will be described.

In this case, image data of a plurality of cells are obtained by the measurement unit 2, for example, multivariate analysis is performed by using the obtained plurality of image data, and a feature amount extraction model that can clearly detect differences between cells is established. Specifically, when the state inference unit 10a receives image data from the measurement unit 2 or the database 4, by performing PCA processing on the image data, the state inference unit 10a extracts, as a feature amount, a principal component vector (unsupervised base) that characterizes the distribution of the image data to generate a feature amount extraction model.

In the state inference system 1a, when image data of unknown target cells are obtained thereafter, it can be inferred, on the basis of the image data of these target cells and the feature amount extraction model, to which cells the unknown target cells have high similarity in comparison with past cells.

In this way, even for the feature amount extraction model generated by the PCA processing, the determination unit 12 can set a threshold value for abnormality determination in the state inference space of the feature amount extraction model on the basis of a certain rule for abnormality determination. In this case, the threshold value is simply defined as a quantity related to the distribution obtained when the image data of each of a plurality of cells is projected onto the state inference space in which the principal component vector is defined.

For example, the determination unit 12 can perform discrimination of the presence or absence of an abnormality by using the threshold value set in the state inference space and a coordinate x obtained when the image data of the target cells are projected onto the state inference space. Alternatively, the degree of abnormality of the target cells may be determined by setting the above-described threshold value to be divided at a plurality of stages. The determination unit 12 outputs a determination result regarding the presence or absence of an abnormality or the degree of abnormality in the analysis to the communication unit 5. As a result, the communication unit 5 can suggest the determination result of the determination unit 12 to the operator or the like.

### (2-3) Other Embodiments Regarding Measurement Time for Characteristics of Biological Sample

### (2-3-1) Case of Measuring Characteristics of Cells During Culturing Process After Start of Culturing Process

In the above-described second embodiment, a case in which image data of cells are obtained by the measurement unit 2 before a culturing process is started has been described; however, the invention is not limited to this. In the second embodiment as well, for example, as shown in 10A in FIG. 10, after the culturing process is started, images of the cells in the middle of being cultured in the culturing process may be captured by the measurement unit 2 to obtain image data.

In this case, in the state inference system 1a, as shown in 10A in FIG. 10, images of a plurality of cells are captured by the measurement unit 2, and image data of the cells are acquired, at a certain time point Sa2 in the middle of performing the culturing process. In this way, in the state inference system 1a, image data at a certain time point Sa2 after a predetermined period has elapsed from the start of culturing continues to accumulate, in regard to a plurality of cells that are currently being cultured by a culturing process.

In a predetermined culturing process, cells of a culture are cultured until the originally planned end timing of culturing, when culturing of the cells is ended, the cells after the end of culturing, which are included in the culture medium, are stained, and the light emitted from the cells is measured to check the differentiation state of the cells from the manifestation state of the light emitted from the cells (Fa1). In this way, the state of each of the cells after culturing is measured, and the actual culturing result of each cell is obtained.

In the state inference system 1a, when the index (actual culture result) indicating the differentiation state of the cells, which is obtained for each cell, is inputted by the operator or the like, the result of the culture state is stored in the database 4. At this time, in the database 4, the image data of each cell measured at a certain time point Sa2 during the culturing process is stored in association with the final culture result obtained when the cells are cultured.

In the state inference unit 10a according to the second embodiment, the image data of each cell measured at a certain time point Sa2 during the culturing process and the final culture state of the cells are read out from the database 4 as sample data to generate a feature amount extraction model.

This feature amount extraction model uses image data of target cells at a certain time point Sa2 (for example, 10^{th} day from the start of culturing) after culturing of the target cells is started by a predetermined culturing process, as the input, and an inference value of the future culture state of the target cells finally obtainable when it is assumed that culturing of the target cells is continued as it is, as the output.

As the feature amount extraction model used herein, similarly to the above-mentioned embodiment, for example, various feature amount extraction models such as PCA, PLS, polynomial regression, Gaussian process regression, and random forest regression; and a feature amount extraction model generated by machine learning of characteristic values (image data) of the cells in the middle of culturing and the culture result thereof, can be applied. When a feature amount extraction model that implements supervised learning is generated, it is desirable to allow the feature amount extraction model to learn the correspondence relation between the image data of a plurality of cells and the culture results of these cells, and characterize the distribution of the image data from the culture results. In this case as well, regarding the feature amount extraction model, unsupervised learning may be applied in addition to supervised learning, as described above. When a feature amount extraction model that implements unsupervised learning is generated, it is desirable to use only the image data of a plurality of cells and allow learning that characterizes the distribution of the cells in a plurality of these image data.

Here, in the state inference unit 10a, for example, in the case of supervised learning, the image data of cells in the middle of being cultured by a culturing process are used as an explanatory variable, and the actual final culture state (differentiation state of the cells at the time of ending culturing) obtained by this culturing process is used as an objective variable, to generate in advance a feature amount extraction model that shows characteristic relevance such as a correlation or an inverse correlation between the image data and the culture state.

As a result, in the state inference system 1a, thereafter, in the middle of culturing undifferentiated target cells in a culturing process, it is possible to infer the future culture result of the target cells that will be finally obtained by culturing, by utilizing the feature amount extraction model, in the middle of culturing before the culturing process is terminated.

For example, in a series of culturing processes for inducing differentiation of cells by culturing and obtaining intended cells, a long time is required to obtain the intended cells. Particularly, a series of culturing processes for inducing differentiation of undifferentiated human stem cells and obtaining intended cells usually takes a time of about three months or longer. In such a culturing process, for example, when it is attempted to obtain an optimum culture result (that is, intended cells) by sequentially optimizing various parameters such as the components of the culture medium and the culture temperature, a method of waiting for the end of the culturing process, analyzing the actual culture result obtained, and determining the next parameters (components of the culture medium to be used, culture temperature, and the like) each time, may be conceived.

However, in this method, for example, in a case where one cycle of the culturing process from the start of culturing to the end of culturing is about 3 months, only about 4 cycles can be carried out at the maximum in one year, and when each parameter of the culturing process gets to be adjusted by trial and error in 4 cycles, the time efficiency is poor.

In contrast, in the state inference system 1a according to the second embodiment, since an inference result for the culture state that will be finally obtained is obtained in the middle of the culturing process on the basis of the feature amount extraction model, by suggesting this inference result to the operator or the like in the middle of culturing, various parameters (components of the culture medium to be used, culture temperature, and the like) of the culturing process can be changed as necessary based on the obtained inference result, without waiting for the end of the culturing process, and a new culturing process can be carried out. In this way, a new culturing process in which the parameters of the culturing process or the culture have been adjusted by trial and error so as to obtain a desired culture result, can be started in the middle of the culturing process that is currently being performed, without waiting for the end of the culturing process, and parameter optimization can be carried out in a time-efficient manner.

In this case, the determination unit 12 determines the state of the target cells on the basis of the state inference result obtained in the state inference unit 10a from the image data of the cells during the culturing process.

### (2-3-2) Case of Measuring Characteristics of Cells Multiple Times During Culturing Process

In the second embodiment as well, similarly to the above-described first embodiment, for example, as shown in 10B in FIG. 10, images of each of the cells may be captured by the measurement unit 2 at a certain time point Sa1 before the culturing process is started and at certain time points Sa2 and Sa3 during the culturing process, and image data may be obtained a plurality of times. In addition, it is also acceptable that images of the cells are not captured by the measurement unit 2 before the culturing process is started, images of each of the cells are captured by the measurement unit 2 only at certain time points Sa2 and Sa3 during the culturing process, and image data of those cells are obtained a plurality of times. That is, image data of the cells may be obtained a plurality of times at certain intervals along the timeline from the start of the culturing process, such as the image data of the cells on the 10^{th} day from the start of the culturing process and the image data of the cells on the 20^{th} day from the start of the culturing process.

In this case, in the state inference system 1a, as shown in 10B in FIG. 10, images of a plurality of cells are captured by the measurement unit 2 at each of a certain time point Sa1 before the culturing process is started and certain time points Sa2 and Sa3 in the middle of carrying out the culturing process, and image data are acquired. In addition, in the state inference system 1a, after the end of the culturing process, the respective culture state of the target cells (differentiation state of the culture) after being actually cultured by using a culture medium is measured (Fa1).

In the state inference unit 10a, a feature amount extraction model is generated each separately on the basis of the image data of the cells at a certain time point Sa1 before the start of the culturing process and each of the image data of the cells at certain time points Sa2 and Sa3 after the start of the culturing process obtained in this way. That is, for example, in the case of supervised learning, the state inference unit 10a uses the image data of the cells before the start of the culturing process as an explanatory variable, and uses the differentiation state of the cells after the end of the culturing process as an objective variable, to generate a feature amount extraction model that shows the relevance between the image data and the culture state. Thereafter, when image data of the target cells in an undifferentiated state are obtained before the start of the culturing process, from these image data, the state inference unit 10a infers the culture state of the target cells (differentiation state of the target cells) that will be obtained in the future when the target cells are cultured by a culturing process, on the basis of the feature amount extraction model.

In the same manner, the state inference unit 10a generates a feature amount extraction model showing the relevance between the image data of the cells at a certain time point Sa2 after the start of the culturing process and the culture state of the cells after the end of the culturing process, and similarly generates a feature amount extraction model showing the relevance between the image data of the cells at a certain time point Sa3 after the start of the culturing process and the culture state of the cells after the end of the culturing process. For target cells in an undifferentiated state during use in culturing, the state inference unit 10a infers the culture state of the target cells (differentiation state of the target cells) that will be obtained in the future when the target cells are cultured by a culturing process, from each of the image data acquired at certain time points (Sa2, Sa3) on the basis of the feature amount extraction model at these certain time points Sa2 and Sa3.

In this way, images of the target cells are captured by the measurement unit 2 at each of a certain time point Sa1 before the culturing process is started and certain time points Sa2 and Sa3 during the culturing process, and while sequentially inferring the culture state of the target cells after the end of the culturing process from the image data of the target cells that change over time, on the basis of the feature amount extraction models corresponding to the certain time points Sa1, Sa2, and Sa3, the culturing process can be executed.

Here, a case in which each feature amount extraction model is generated by supervised learning has been described; however, the invention is not limited to this, and each feature amount extraction model may also be generated by unsupervised learning according to the above-described section "(2-2) Unsupervised Generation of Feature Amount Extraction Model".

### (2-3-3) Other Embodiments of Inferring Culture State of Cells in Middle of Culturing Before End of Culturing Process

In the above-described second embodiment, a case of inferring the culture state of the target cells included in the culture medium (for example, differentiation state of the target cells) after the culturing process is ended (after the end timing F) has been described; however, the invention is not limited to this. In the above-described second embodiment, for example, as shown in 11A in FIG. 11, the culture state of the target cells at a certain time point Fa2 in the middle of culturing before the culturing process is ended, may be inferred.

In this case, in the state inference system 1a, as shown in 11A in FIG. 11, images of a plurality of cells are captured by the measurement unit 2 at a certain time point Sa1 before the culturing process is started, and the image data are acquired. In addition, in the state inference system 1a, the culture state of the cells (differentiation state of the cells at that time point) in the middle of being actually cultured is measured at a certain time point Fa2 before the culturing process is ended.

In the state inference unit 10a, a feature amount extraction model is generated by supervised learning on the basis of the image data of undifferentiated cells before the start of the culturing process and the culture state of the cells in the middle of culturing, which are obtained in this way. That is, the state inference unit 10a uses the image data of the cells before the start of the culturing process as an explanatory variable and uses the culture state of the cells at a certain time point Fa2 before the end of the culturing process as an objective variable, to generate a feature amount extraction model that characterizes the relevance between the image data and the culture state. Thereafter, when image data on the target cells in an undifferentiated state are obtained before the start of the culturing process, the state inference unit 10a can infer the culture state of the target cells in the middle of culturing (differentiation state of the target cells), which will be obtained in the future when the target cells are cultured by the culturing process on the basis of the feature amount extraction model.

When a feature amount extraction model is generated by supervised learning, for example, it is desirable that the data indicating the differentiation state of each cell that has been actually cultured to a certain time point Fa2 before the end of the culturing process, as an index, is applied as an objective variable. In addition, the characteristic state itself of each cell in the image data (that is, the image data itself obtained by imaging the cell, without indicating the differentiation state of an actually cultured cell with an "index") obtained by staining each cell that has been actually cultured to the time point Fa2 with a staining substance and imaging the cell with the measurement unit 2, which is a microscope apparatus, may also be applied as an objective variable.

Here, as shown in 11A in FIG. 11, a case in which images of a plurality of cells in an undifferentiated state are captured by the measurement unit 2 at a certain time point Sa1 before the culturing process is started, and image data are acquired, has been described; however, the invention is not limited to this. In this second embodiment as well, for example, as shown in 11B in FIG. 11, images of a plurality of cells may be captured by the measurement unit 2 at a certain time point Sa2 in the middle of performing the culturing process, and image data may be acquired.

In this case, the state inference unit 10a uses the image data of the cells at a certain time point Sa2 in the middle of performing the culturing process as an explanatory variable, and uses the culture state of the cells at a certain time point Fa2 before the end of the culturing process as an objective variable, to generate a feature amount extraction model that characterizes the relevance between the image data and the culture state. Thereafter, when image data obtained by imaging the target cells at a certain time point Sa2 in the middle of performing the culturing process are obtained, the state inference unit 10a can infer the culture state of the target cells (differentiation state of the target cells) at a certain time point Fa2 in the middle of culturing, which will be obtained in the future, from the mass image data on the basis of the feature amount extraction model.

Even in this second embodiment, as shown in 11C in FIG. 11, images of the cells may be captured by the measurement unit 2 at each of a certain time point Sa1 before the culturing process is started and certain time points Sa2 and Sa3 during the culturing process, and image data may be obtained a plurality of times. In addition, it is also acceptable that images of the cells are not captured by the measurement unit 2 before the culturing process is started, images of the cells are captured by the measurement unit 2 only at each of the time points Sa2 and Sa3 during the culturing process, and image data are obtained a plurality of times.

The state inference unit 10a uses, for example, the image data of the cells before the start of the culturing process as an explanatory variable, and uses the culture state of the cells included in the culture medium at a certain time point Fa2 before the culturing process is ended, as an objective variable, to generate a feature amount extraction model that characterizes the relevance between the image data and the culture state. Thereafter, when image data of undifferentiated target cells before the start of the culturing process are obtained, from these image data, the state inference unit 10a can infer the culture state of the target cells (differentiation state of the target cells) at a certain time point Fa2 in the middle of culturing, which will be obtained in the future, on the basis of the feature amount extraction model.

The state inference unit 10a generates a feature amount extraction model that characterizes the relevance between the image data of the cells at a certain time point Sa2 after the start of the culturing process and the culture state of the cells included in the culture medium at a certain time point Fa2 before the culturing process is ended, and similarly generates a feature amount extraction model that characterizes the relevance between the image data of the cells at a certain time point Sa3 after the start of the culturing process and the culture state of the cells included in the culture medium at a certain time point Fa2 before the culturing process is ended. Thereafter, when image data are obtained at each of certain time points (Sa2, Sa3) for target cells during culturing, the state inference unit 10a can infer, from the image data, each of the culture states of the target cells (differentiation state of the target cells) at a certain time point Fa2 in the middle of culturing, which will be obtained in the future, on the basis of the feature amount extraction models at the corresponding certain time points Sa2 and Sa3.

Even in each of the sections "(1-9) Optimization Inference Processing of Culture Parameters" and "(1-10) State Inference System Inferring Culture Time", as described above, by replacing the "mass spectrometry data" (or measurement vector) that are acquired through the measurement unit 2 with "image data", processing of each of these sections can be applied.

For example, in "(1-9) Optimization Inference Processing of Culture Parameters", the state inference unit 10c uses image data of a plurality of cultures and culture parameters related to the culturing conditions for a culturing process using the cultures, as input data, to generate a feature amount extraction model for culture parameter inference that characterizes the distributions of the image data and the culture parameters.

More specifically, the state inference unit 10c generates a feature amount extraction model for culture parameter inference, which uses the image data of cells obtained by the measurement unit 2, a culture parameter a before correction, which serves as a reference, and correction candidate values Δa0, Δa1, Δa1, and Δa1, as the input (explanatory variables), and uses the culture state (here, differentiation state) of the cells after culturing as the output (objective variable), by using a neural network or the like.

The state inference unit 10c infers the culture state (differentiation state) of target cells by inputting image data of the target cells obtained through the measurement unit 2, the culture parameter a, and a plurality of correction candidate values Δa of the culture parameter as input data, into the above-mentioned feature amount extraction model for culture parameter inference, and performing analysis.

The state inference unit 10c outputs a plurality of inference results obtained by changing each of the correction candidate values Δa, to the culture parameter optimization inference unit 15. The culture parameter optimization inference unit 15 selects an inference result that is the closest to the result of the optimal culture state that has been set in advance among the plurality of inference results obtained in the state inference unit 10c, and specifies the correction candidate value Δa of the culture parameter a set with the selected inference result.

In " (1-10) State Inference System Inferring Culture Time", for example, in the case of supervised learning, a feature amount extraction model is generated by the state inference unit 10a by using image data of a plurality of cells obtained by the measurement unit 2 at a certain time point as an explanatory variable, and using the culture time in which, when cells are actually cultured by a culturing process, the culture reaches a desired culture state, as an objective variable. In such a feature amount extraction model, the appearance feature of cells can be analyzed, which affects the culture time in which, when a culture is cultured by using a culture medium, the culture reaches a desired culture state (hereinafter, simply referred to as culture time).

In the second embodiment as well, regarding supervised learning of other feature amount extraction models, for example, the image data of a plurality of cells, each culture time for these cells, and a truth label indicating whether the culture time is a desired result (for example, an accuracy indicating whether the culture time is a desired culture time) are used, and when the image data of the cells and the culture time thereof are learned, this learning may be carried out by associating the image data of the cells and the culture time with the truth label, so that the distribution of the mass spectrometry data may be characterized.

Regarding the feature amount extraction model, for example, various feature amount extraction models such as PCA, PLS, polynomial regression, Gaussian process regression, and random forest regression can be applied. As the feature amount extraction model, for example, a machine learning model generated by machine learning of the characteristic values (mass spectrometry data) of the culture medium and the results of the culture time, may also be applied.

As the machine learning model, for example, a neural network (for example, a convolutional neural network (CNN) or a Bayesian neural network (BNN)) can be applied. In addition, the feature amount extraction model may involve supervised learning as described above, as well as unsupervised learning. The details in the case of generating the feature amount extraction model by unsupervised learning will be described below; however, when the feature amount extraction model is generated by unsupervised learning, it is desirable to use the image data of a plurality of cells and allow learning that characterizes the distribution of a plurality of these image data.

In the above-described section "(1-10-2) Regarding State Inference System Shown in FIG. 12", optimal culture parameters are inferred on the basis of the inference result of the culture time by using mass spectrometry data; however, even with regard to this, it is needless to say that it is possible to apply the "mass spectrometry data" as the "image data".

That is, in this case, the state inference unit 10c can infer the culture time taken until the culture reaches a desired culture state (differentiation state) by inputting image data of the target cells measured through the measurement unit 2, a culture parameter a, and a plurality of correction candidate values Δa of the culture parameter as input data into a feature amount extraction model for culture parameter inference and performing analysis.

### (3) Third Embodiment

In the above-described first embodiment, a case in which, for example, a mass spectrometer that measures the mass spectrometry data of a plurality of biological samples and a target biological sample is applied as a measurement unit that measures the characteristics of a plurality of biological samples and a target biological sample, has been described; however, in the third embodiment, with regard to a case of applying a measurement apparatus that measures the impedances of a plurality of biological samples and a target biological sample, an outline will be described below.

In this case, for example, in the state inference system 1a, any of a culture medium, a culture target cultured by using the culture medium, a culture medium extract extracted from the culture medium, and a culture target extract extracted from the culture target, are used as a biological sample and a target biological sample, and the impedances of a plurality of biological samples at a certain time point and the impedance of at least one target biological sample of the same type as the biological sample at the time point are measured as characteristic values with the measurement unit 2.

In the state inference system 1a, the impedances of the plurality of biological samples acquired through the measurement unit 2 are used as input data, a feature amount extraction regression model that characteristics the distribution of these input data is generated, and this is stored in the database 4. Then, in the state inference system 1a, the culture state of the culture is inferred by the state inference unit 10a from the impedance of the target biological sample on the basis of the above-described feature amount extraction model.

In such third embodiment, in each of the sections "(1-1) Configuration of State Inference System", "(1-2) State Inference Unit", "(1-3) Determination Unit", "(1-4) Measurement Target Optimization Inference Unit", "(1-5) Action and Effects", "(1-6) Unsupervised Generation of Feature Amount Extraction Model", "(1-7) Other Embodiments Regarding Measurement Time for Characteristics of Biological Samples", "(1-8) Other Embodiments of Inferring Culture State of Culture in Middle of Culturing Before End of Culturing Process", "(1-9) Optimization Inference Processing of Culture Parameters", and "(1-10) State Inference System Inferring Culture Time" as described in the above-described "(1) First Embodiment", processing of each of these sections can be applied by replacing the "mass spectrometry data" (or measurement vector) acquired through the measurement unit 2 with the "impedance".

For example, in the above-described section " (1-4) Measurement Target Optimization Inference Unit", when an inference result of the culture state (state inference result) of a culture is obtained in the state inference unit 10a from the impedance of the target culture medium before the start of the culturing /process on the basis of a feature amount extraction model, the state inference result is outputted from the state inference unit 10a to the measurement target optimization inference unit 14.

The measurement target optimization inference unit 14 analyzes the state inference result obtained in the state inference unit 10a and infers an additive that can maximize the yield of the culture per unit volume of the culture medium included in the target culture medium after use in culturing, when added to the target culture medium before use in culturing. The measurement target optimization inference unit 14 allows the operator or the like to correct the components of the target culture medium to be used in the culturing process, by adding the additive that has been inferred (also referred to as inferred additive) to the target culture medium.

In "(1-9) Optimization Inference Processing of Culture Parameters", for example, the impedances of a plurality of biological samples and culture parameters related to the culturing conditions for a culturing process using the biological samples are used as input data, and the culture state is inferred from the input data of a target biological sample on the basis of a feature amount extraction model that characterizes the impedances as characteristic values and the distribution of the culture parameters.

In "(1-10) State Inference System Inferring Culture Time", in the state inference system 1a, the impedances of a plurality of biological samples acquired through the measurement unit 2 are used as input data, a feature amount extraction regression model that characterizes the distribution of these input data is generated, and this is stored in the database 4. Then, in the state inference system 1a, the culture time in which the culture reaches a predetermined culture state is inferred from the impedance of the target biological sample on the basis of the above-described feature amount extraction model by the state inference unit 10a.

In addition, as the measurement unit, a Raman spectrometer, chromatography, a digital PCR measurement apparatus, a nuclear magnetic resonance apparatus, an antibody quantification kit using an antibody quantification method, and a nucleic acid sequencing apparatus may be applied. As the nucleic acid sequencing apparatus, for example, nucleic acid sequencing apparatuses that use known DNA sequencing methods such as Sanger sequencing and Next Generation Sequencing (NGS) may be mentioned; however, the examples are not limited to these.

In addition, a flow cytometer that will be described in the fourth embodiment may also be applied as the measurement unit 2.

In this case, for any one among a Raman spectrum obtained from a Raman spectrometer, a chromatogram obtained from chromatography, the amount of a specific substance obtained from a digital PCR measurement apparatus, analytical data obtained from a nuclear magnetic resonance apparatus, the amount of a specific antigen obtained from an antibody quantification kit, and gene sequence analysis data obtained from a nucleic acid sequencing apparatus, by replacing the "mass spectrometry data" (or measurement vector) acquired through the measurement unit 2 in each of the sections "(1-1) Configuration of State Inference System", "(1-2) State Inference Unit", "(1-3) Determination Unit", "(1-4) Measurement Target Optimization Inference Unit", "(1-5) Action and Effects", "(1-6) Unsupervised Generation of Feature Amount Extraction Model", "(1-7) Other Embodiments Regarding Measurement Time for Characteristics of Biological Samples", "(1-8) Other Embodiments of Inferring Culture State of Culture in Middle of Culturing Before End of Culturing Process", "(1-9) Optimization Inference Processing of Culture Parameters", "(1-10) State Inference System Inferring Culture Time", and "(1-11) Others" as described in the above-described "(1) First Embodiment", with a "Raman spectrum" or "gene sequence analysis data" for example, processing of each of these sections can be applied.

Thus, in the embodiment, as the state inference unit, for example, a state inference unit that infers the yield of a culture or infers the culture time in which a culture reaches a predetermined yield, from the input data of the culture medium at a certain time point before the end of culturing on the basis of a feature amount extraction model, has been mainly described; however, as described above, the invention is not limited to the "yield of a culture", and the "quality of a culture", the "yield (quality) of a culture extract extracted from a culture", or the "yield (quality) of a component in a culture medium secreted by a cultured culture" can be applied as the "culture state of a culture".

For example, the state inference unit may be a state inference unit that infers the quality of a culture or infers the culture time in which a culture reaches a predetermined quality, from the input data obtained from the culture medium or the culture at a certain time point before the end of culturing on the basis of a feature amount extraction model.

The state inference unit may also be a state inference unit that infers the yield (quality) of a culture extract extracted from a culture or infers the culture time in which a culture extract reaches a predetermined yield (quality), from the input data obtained from the culture medium or the culture at a certain time point before the end of culturing on the basis of a feature amount extraction model. Furthermore, the state inference unit may be a state inference unit that infers the yield (quality) of a component in the culture medium secreted by a cultured culture or infers the culture time in which a component in the culture medium secreted by the culture reaches a predetermined yield (quality), from the input data obtained from the culture medium or culture at a certain time point before the end of culturing on the basis of a feature amount extraction model.

### (4) Fourth Embodiment

### (4-1) State Inference System According to Fourth Embodiment

Next, the fourth embodiment will be described. A state inference system according to the fourth embodiment uses a measurement result obtained by measuring the characteristic values of a culture at a certain time point at the measurement unit, as input data, and infers, from the input data, which culture state will be obtained in the future from among a plurality of types of culture states obtainable when the culture is cultured according to a culturing process, on the basis of a feature amount extraction model. Then, the state inference system determines the next processing step that will be performed on the culture at a certain time point, according to the inferred type of culture state of the culture.

Here, FIG. 13 is a schematic view for describing the types of the culture state of culture A, which is inferred in the state inference system according to the fourth embodiment, and shows an example in which culture A is induced to differentiate into culture B, culture C, or non-intended target cells by culturing. Here, a case in which when culture A (for example, human iPS cells (hereinafter, simply referred to as iPS cells)) shown in step S10 is cultured in a culture medium including a predetermined cytokine, depending on the influence of the state or quality of the culture A, the quality of the culture medium, or the like, the culture A reaches one of three ways of culture states, that is, the culture A is induced to differentiate into culture B of paraxial mesodermal cells (step S11), the culture A is induced to differentiate into culture C of lateral plate mesodermal cells (step S12), or the culture A becomes, for example, non-intended target cells such as other different cells, dead cells, or cells in a low-quality state (step S13), will be described as an example.

In the fourth embodiment, it is assumed that the optimal culturing conditions for inducing differentiation of the culture A into the culture B and the optimal culturing conditions for inducing differentiation of the culture A into the culture C are culturing conditions different from each other. In the state inference system according to the fourth embodiment, any one of a processing step of culturing the culture A under optimal culturing conditions for inducing differentiation into culture B, a processing step of culturing the culture A under optimal culturing conditions for inducing differentiation into culture C, and a processing step of discarding the culture A is appropriately selected on the basis of the inference result for the culture A, and an optimal processing step according to the future culture state of the culture A is executed. Here, the culturing conditions also include various parameters (culture parameters) related to the culturing conditions, for example, the culture temperature and culture time of the culturing process, the pH of the culture medium, and the stirring speed of the culture medium, as well as the type or quality of the culture medium.

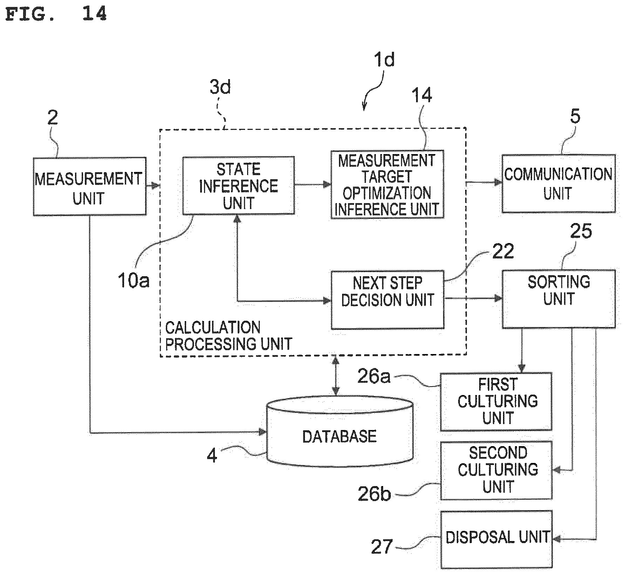

FIG. 14 in which the same configurations as those in FIG. 1 are assigned with the same reference numerals, is a block diagram showing the configuration of a state inference system 1d according to the fourth embodiment. In the fourth embodiment, an example of culture will be shown as an example of biological sample. In addition, the fourth embodiment shows an example of applying a flow cytometer using a flow cytometry method as the measurement unit 2.

In the measurement unit 2, for example, the culture A at a certain time point when culturing is not completed, such as before the start of culturing or immediately after the start of culturing, is suspended in a liquid, and while the liquid is caused to flow into a flow path such that the culture A is lined up in a line, laser light is applied to be reflected on each of culture A, and the reflected light such as scattered light or fluorescence is measured. The measurement unit 2 obtains the respective characteristic values for each culture A by analyzing the measurement results of reflected light, and the analytical data showing the characteristic values measured for each culture A are generated as input data of each culture A.

The state inference system 1d according to the fourth embodiment measures, for example, each of a plurality of cultures A at a certain time point by using the measurement unit 2 and generates a feature amount extraction model on the basis of the obtained analytical data. Here, the analytical data are the intensity of reflected light, the intensity of scattered light, the intensity of fluorescence, and the like, which are measured when the culture A is analyzed by using a flow cytometer, which is the measurement unit 2, and the analytical data may serve as characteristic values of the culture A. Once a feature amount extraction model is obtained, the state inference system 1d can measure the culture A, for which it has been undecided what kind of culture state the culture A will be actually induced to differentiate into, with the measurement unit 2, and infer what kind of culture state the culture A will reach in the future when the culture A, for which the future culture state is not yet decided at the present time point, is continued to be cultured as it is, on the basis of the obtained analytical data and the feature amount extraction model. In this way, the culture A whose culture state is inferred by using the generated feature amount extraction model is referred to as "target culture".

The measurement unit 2, which is a flow cytometer, measures the characteristic values of each of a plurality of cultures A and outputs the obtained measurement results to a calculation processing unit 3d and the database 4. The calculation processing unit 3d has the state inference unit 10a, the measurement target optimization inference unit 14, and a next step decision unit 22. Since the measurement target optimization inference unit 14 is similar to that of the above-described embodiments, description thereof will not be repeated here, and the following description will be focused on the state inference unit 10a and the next step decision unit 22. In this case, in the state inference unit 10a, when a feature amount extraction model is generated, each of the measurement results of a plurality of cultures A from the measurement unit 2 or the database 4, which are obtained by using a flow cytometer (measurement unit 2), is inputted as analytical data.

In the state inference unit 10a according to the present embodiment, a feature amount extraction model is generated by, for example, performing multivariate analysis by using the analytical data of the plurality of the cultures A measured using the measurement unit 2 at a certain time point, and a result of the culture state (hereinafter, also referred to as a culture result) when each of the cultures A is actually cultured by using a predetermined culture medium. In the present embodiment, regarding the culture state when the culture A is cultured, there are a plurality of states, such as a case in which the culture A becomes culture B, a case in which the culture A becomes culture C, and a case in which the culture A becomes non-intended target cells, and therefore, here, an example in which a feature amount extraction model for inferring each of culture state is generated for each of the culture states will be described as an example. An example of inferring a plurality of types of culture states on the basis of one feature amount extraction model will be described below.

That is, the state inference unit 10a generates each of a feature amount extraction model for culture B, which infers whether culture B will be obtained by culturing the culture A, a feature amount extraction model for culture C, which infers whether culture C will be obtained by culturing the culture A, and a feature amount extraction model for non-intended target cells, which infers whether non-intended target cells will be obtained by culturing the culture A. Such feature amount extraction models can analyze the state, quality, and the like of the culture A, which will affect the culture result obtained when the culture A is cultured by using a culture medium.

The feature amount extraction model generated for each of the culture results obtained by culturing the culture A may be generated by supervised learning or may be generated by unsupervised learning. For example, Regarding a feature amount extraction model generated by supervised learning, it is desirable to apply a regression model or the like, which uses the analytical data obtained as a result of measuring a target culture A at a certain time point with the measurement unit 2 as the input, and uses the state inference result that infers the future culture state that is finally obtained when it is assumed that culturing of the target culture A is continued as it is, as the output.

In this case, for example, when a feature amount extraction model for culture B is generated in the state inference unit 10a by supervised learning, the feature amount extraction model is allowed to learn the correspondence relation between a plurality of analytical data obtained by measuring each of a plurality of cultures A at a certain time point with the measurement unit 2, and the culture result obtained when each of these cultures A is actually cultured to obtain culture B, and the distribution of the analytical data is characterized from the culture results obtained when the culture A becomes the culture B.

Similarly, when a feature amount extraction model for culture C is generated in the state inference unit 10a by supervised learning, the feature amount extraction model is allowed to learn the correspondence relation between a plurality of analytical data obtained by measuring each of a plurality of cultures A at a certain time point with the measurement unit 2, and the culture result obtained when each of these cultures A is actually cultured to obtain culture C, and the distribution of the analytical data is characterized from the culture results obtained when the culture A becomes the culture C.

Similarly, when a feature amount extraction model for non-intended target cells is generated in the state inference unit 10a by supervised learning, the feature amount extraction model is allowed to learn the correspondence relation between a plurality of analytical data obtained by measuring each of a plurality of cultures A at a certain time point with the measurement unit 2, and the culture result obtained when each of these cultures A is actually cultured to obtain non-intended target cells, and the distribution of the analytical data is characterized from the culture results obtained when the culture A becomes the non-intended target cells.

In addition to that, regarding supervised learning of the feature amount extraction model for culture B, for example, the analytical data of a plurality of cultures A, each culture result obtained when these cultures A are actually cultured, and a truth label indicating whether the culture result is the desired culture B (for example, an accuracy indicating whether the culture result is the desired culture B) are used, and when the analytical data of the culture A and the culture result thereof are learned, this learning may be carried out by associating the analytical data and the culture result with the truth label indicating that the culture A has become the culture B, so that the distribution of the analytical data may be characterized.

In this case, supervised learning of a feature amount extraction model for culture C (or for non-intended target cells) is carried out in the same manner, the analytical data of a plurality of cultures A, each culture result obtained when these cultures A are actually cultured, and a truth label indicating whether the culture result is the desired culture C (or the non-intended target cells) (for example, an accuracy indicating whether the culture result is the desired culture B (or the non-intended target cells)) are used, and when the analytical data of the culture A and the culture result thereof are learned, this learning is carried out by associating the analytical data and the culture result with the truth label indicating that the culture A has become the culture C (or the non-intended target cells), so that the distribution of the analytical data is characterized.

As the feature amount extraction model, for example, various feature amount extraction models such as principal component analysis (PCA), partial least squares (PLS), polynomial regression, Gaussian process regression, and random forest regression can be applied. As the feature amount extraction model, for example, a machine learning model generated by machine learning of the characteristic values (analytical data obtained using a flow cytometer) of the culture A and the culture result, may also be applied.

As the machine learning model, similarly to the above-described embodiments, for example, a neural network (CNN or BNN) can be applied in addition to the above-described polynomial regression, Gaussian process regression, and random forest regression. When each of a feature amount extraction model for culture B, a feature amount extraction model for culture C, and a feature amount extraction model for non-intended target cells is generated by unsupervised learning, similarly to the above-described embodiments, it is desirable to use the analytical data of a plurality of cultures A and allow learning that characterizes the distribution of a plurality of these analytical data for each of the culture result for the culture B, the culture C, or the non-intended target cells.

Specifically, in a case where a feature amount extraction model is generated by unsupervised learning, the state inference unit 10a approximates the distribution of the analytical data group of a plurality of cultures A that have become culture B by culturing and the distribution of the analytical data group of a plurality of cultures A that have become culture C by culturing, for example, by multi-dimensional Gaussian distribution. By performing threshold value calculation according to the Mahalanobis distance in the state inference space to the distribution center of the multi-dimensional Gaussian distribution generated by the above-described processing, a feature amount extraction model that identifies the non-intended target cells and others (here, culture B and culture C) can be established. Next, by subjecting the distribution in the state inference space of the analytical data group of a plurality of cultures A that have become the culture B by culturing, and the distribution in the state inference space of the analytical data group of a plurality of cultures A that have become the culture C, for example, to clustering by a k-means method, clusters corresponding to the cultures B and clusters corresponding to the cultures C can be obtained, and this is a feature amount extraction model that identifies the culture B and the culture C.

When the future culture state of a target culture A is inferred, the state inference unit 10a inputs the analytical data of the target culture A into the feature amount extraction model for culture B, and outputs the inference result that infers whether the culture state of the target culture A will be the culture B in the future when culturing of the target culture A is continued as it is, on the basis of the feature amount extraction model for culture B. Similarly, the state inference unit 10a also inputs, for example, the analytical data of the target culture A into the feature amount extraction model for culture C, and outputs the inference result that infers whether the culture state of the target culture A will be the culture C in the future when culturing of the target culture A is continued as it is, on the basis of the feature amount extraction model for culture C. Furthermore, the state inference unit 10a inputs, for example, the analytical data of the target culture A into the feature amount extraction model for non-intended target cells, and outputs the inference result that infers whether the culture state of the target culture A will be the non-intended target cells in the future when culturing of the target culture A is continued as it is, on the basis of the feature amount extraction model for non-intended target cells.

Among the inference result obtained from the feature amount extraction model for culture B, the inference result obtained from the feature amount extraction model for culture C, and the inference result obtained from the feature amount extraction model for non-intended target cells, the state inference unit 10a selects the inference result with the highest accuracy obtainable with inference results, as an inference result that the inference result is the future culture state when culturing the target culture A is continued.

In the above-described fourth embodiment, a case in which each of a feature amount extraction model for culture B, a feature amount extraction model for culture C, and a feature amount extraction model for non-intended target cells is generated, input data are inputted into each of the feature amount extraction models to obtain an inference result for each of the feature amount extraction models, has been described; however, the invention is not limited to this. For example, it is desirable that one feature amount extraction model is generated, input data are inputted into the feature amount extraction model, and an inference result that infers the future culture state of the culture A on the basis of the one feature amount extraction model is obtained.

For example, in a case where one feature amount extraction model is generated by supervised learning, the state inference unit 10a allows learning of the correspondence relation between a plurality of analytical data obtained by measuring each of a plurality of cultures A at a certain time point using the measurement unit 2, and a culture result in which each of these cultures A is actually cultured to obtain culture B, culture C, or non-intended target cells, and generates one feature amount extraction model that characterizes the distribution of the analytical data from these culture results.

For example, in a case where one feature amount extraction model is generated by unsupervised learning, in the state inference unit 10a, the analytical data of the culture A that becomes culture B, the analytical data of the culture A that becomes culture C, and the analytical data of the culture A that becomes non-intended target cells, are clustered by a k-means method, optimization of classification of a plurality of these analytical data without labels is performed in the state inference space in which the feature amount is defined, and then the obtained clusters are labeled with culture B, culture C, and non-intended target cells (assigning identical values such as straight lines and curves that can be identified stepwise) by the operator or the like to generate a feature amount extraction model. As a result, the state inference unit 10a can project the analytical data of the target culture A onto the state inference space of the feature amount extraction model, and infer whether the culture state of the target culture A will be the culture B, the culture C, or the non-intended target cells in the future when culturing the target culture A is continued, by using an identification value (a label or the like) as a guide.

When an inference result that infers the future culture state of the target culture A is obtained, the state inference unit 10a outputs this to the next step decision unit 22. Among a processing step of culturing the target culture A under optimal culturing conditions for inducing differentiation of the target culture A into the culture B (hereinafter, also referred to as a processing step for culture B), a processing step of culturing the target culture A under optimal culturing conditions for inducing differentiation of the target culture A into the culture C (hereinafter, also referred to as a processing step for culture C), and a processing step of discarding the target culture A (hereinafter, also referred to as a disposal processing step), the next step decision unit 22 determines any one processing step on the basis of the inference results for the target culture A and outputs the determination result as the information on next step to a sorting unit 25.

Specifically, when the next step decision unit 22 receives an inference result that the future culture state of the target culture A is the culture B from the state inference unit 10a, the next step decision unit 22 determines a processing step for culture B, and when the next step decision unit 22 receives an inference result that the future culture state of the target culture A is the culture C from the state inference unit 10a, the next step decision unit 22 determines a processing step for culture C. In addition, when the next step decision unit 22 receives an inference result that the future culture state of the target culture A is the non-intended target cells from the state inference unit 10a, the next step decision unit 22 determines a disposal processing step.

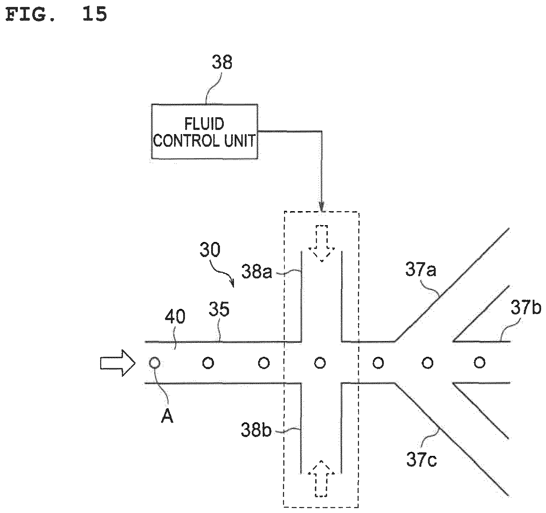

The sorting unit 25 is configured to automatically sort the target culture A on the basis of the information on next step received from the next step decision unit 22, and send out the target culture A to any of a first culturing unit 26a, a second culturing unit 26b, or a disposal unit 27. As shown in FIG. 15, the sorting unit 25 is provided with a substrate 30 on which a main flow channel 35, sub-flow channels 38a and 38b, and branch flow channels 37a, 37b, and 37c are formed.

The main flow channel 35 formed in the substrate 30 is configured such that the upstream side communicates with a flow channel of a flow cytometer, which is the measurement unit 2, and a liquid 40 containing a plurality of cultures A that are lined up in a line, flows in through the flow channel of the flow cytometer. The flow rate of the liquid 40 flowing into the main flow channel 35 from the flow channel of the flow cytometer is controlled by a syringe pump, a rotary pump, a centrifugal pump, or a pneumatic pump, which are not shown in the diagram.

The branch flow channels 37a, 37b, and 37c communicate with the main flow channel 35 on the downstream side, and one end of each of the sub-flow channels 38a and 38b communicates between the flow channel of the flow cytometer and the branch flow channels 37a, 37b, and 37c. The sub-flow channels 38a and 38b are disposed to face each other so as to orthogonally intersects the flow direction of the main flow channel 35. The sub-flow channels 38a and 38b adjusts the flow direction of the liquid 40 in the main flow channel 35 by controlling the inflow of the liquid for control into the main flow channel 35, and allows each of the culture A contained in the liquid 40 to flow out to each of optimal branch flow channels 37a, 37b, and 37c. The inflow of the liquid for control from the sub-flow channels 38a and 38b to the main flow channel 35 is controlled by a fluid control unit 38 such as a syringe pump, a rotary pump, a centrifugal pump, or a pneumatic pump, which is provided at the sub-flow channels 38a and 38b.

Here, the downstream of the branch flow channel 37a communicates with the first culturing unit 26a that executes the processing step for culture B, the downstream of the branch flow channel 37b communicates with the second culturing unit 26b that executes the processing step for culture C, and the downstream of the branch flow channel 37c communicates with the disposal unit 27 that executes the disposal processing step.

In the sorting unit 25, the fluid control unit 38 receives the information on next step from the next step decision unit 22, the inflow of the liquid for control from the sub-flow channels 38a and 38b to the main flow channel 35 is adjusted by the fluid control unit 38 according to the information on next step, and the target culture A that has obtained the information on next step is caused to flow out to the branch flow channels 37a, 37b, and 37c corresponding to the information on next step.

For example, when the target culture A that has been decided to go through the processing step for culture B from the next step decision unit 22 flows through in the main flow channel 35, the sorting unit 25 controls the inflow of the liquid from the sub-flow channels 38a and 38b to the main flow channel 35 through the fluid control unit 38, leads the target culture A to the branch flow channel 37a, and guides the target culture A to the first culturing unit 26a through the branch flow channel 37a. As a result, the target culture A that has been decided to go through the processing step for culture B is cultured by the first culturing unit 26a under optimal culturing conditions for inducing differentiation into the culture B.

When the target culture A that has been decided to go through the processing step for culture C from the next step decision unit 22 flows through in the main flow channel 35, the sorting unit 25 controls the inflow of the liquid from the sub-flow channels 38a and 38b to the main flow channel 35 by the fluid control unit 38, leads the target culture A to the branch flow channel 37b, and guides the target culture A to the second culturing unit 26b through the branch flow channel 37b. As a result, the target culture A that has been decided to go through the processing step for culture C is cultured by the second culturing unit 26b under optimal culturing conditions for inducing differentiation into the culture C.

When the target culture A that has been decided to go through the disposal processing step from the next step decision unit 22 flows through in the main flow channel 35, the sorting unit 25 controls the inflow of the liquid from the sub-flow channels 38a and 38b to the main flow channel 35 by the fluid control unit 38, leads the target culture A to the branch flow channel 37c, and guides the target culture A to the disposal unit 27 through the branch flow channel 37c. As a result, the target culture A that has been decided to go through the disposal processing step is discarded by the disposal unit 27.

### (4-2) Action and Effects

With regard to the above-described configuration, the state inference system 1d according to the fourth embodiment uses the characteristic values of a plurality of cultures A measured in the measurement unit 2 as the input data, and infers, in the state inference unit 10a, which culture state (culture B, culture C, or non-intended target cells) among a plurality of types of culture states the culture A will reach in the future when the culture A is cultured, from the characteristic values of the target culture A on the basis of a feature amount extraction model that characterizes the distribution of those input data. In this way, in the state inference system 1d as well, since it can be inferred in advance whether a desired culture state can be obtained from a target culture A in the same manner as in the above-described embodiments, the time required for making a proper decision on the target culture A can be shortened. As a result, the efficiency of bioproduction or research and development using the culture A can be improved.

In addition to this, in the state inference system 1d according to the fourth embodiment, when the culture A is cultured, only a culture A that will be a specific culture state in the future can be distinguished in an early stage, and culturing can be carried out under optimal culturing conditions for bringing only the distinguished culture A into a specific culture state.

For example, in a case where differentiation-induced culture of iPS cells is performed to obtain intended cells (for example, retina, nerves, red blood cells, or heart muscle), it can be inferred in advance whether iPS cells will become the intended cells when iPS cells are cultured, without actually culturing the iPS cells, by using the iPS cells as the culture A and using the above-mentioned state inference system 1d. In addition, in a case where iPS cell-derived myocardial cells as intended cells are obtained from iPS cells sequentially through mesodermal cells and myocardial progenitor cells by culturing, since it can be inferred whether iPS cells will first become mesodermal cells by culturing, in an early stage before culturing or in the middle of culturing of the iPS cells by using the above-mentioned state inference system 1d, the time required for making a proper decision on the iPS cells can be shortened to a large extent.

Therefore, in the state inference system 1d, when iPS cells are cultured to produce iPS cell-derived myocardial cells, which are intended cells, iPS cells that can produce the intended cells with a high probability in the future, and iPS cells that may not be able to produce intended cells even when cultured, can be distinguished, and since only those iPS cells that can produce intended cells with a high probability in the future can be sorted, and culturing thereof can be continued, that much extra culturing operation can be omitted so that the culturing operation can be performed efficiently.

### (4-3) Other Embodiments

In the above-described fourth embodiment, a case in which cells obtainable by culturing culture A (culture B or culture C) are inferred as a future culture state of the culture A from measurement results of the culture A on the basis of a feature amount extraction model, has been described; however, the invention is not limited to this. For example, various blood cells such as red blood cells, white blood cells, and platelets, which are obtained by culturing the culture A, or substances and components other than cells, may be inferred as the future culture state of the culture A from measurement results for the culture A on the basis of a feature amount extraction model.

In the above-described embodiments, as an example of generating a feature amount extraction model, for example, a case in which a feature amount extraction model is generated by utilizing a measurement result of culture A obtained by using the measurement unit 2 and a culture result in which the culture A becomes culture B, culture C, or non-intended target cells when the culture A is cultured, has been described; however, the invention is not limited to this, and the feature amount extraction model may be generated by using only the culture result in which the culture A becomes culture B and culture C when cultured, without using the culture result in which the culture A becomes non-intended target cells when cultured. In this case, when a measurement result obtained by measuring the characteristic values of a target culture A by the measurement unit 2 is used as the input data, and the future culture state of the target culture A is inferred from the input data on the basis of a feature amount extraction model, an inference result that the culture A does not become culture B and culture C is outputted as an inference result that the culture A becomes non-intended target cells.

In the above-described embodiments, a measurement result of a target culture A obtained by the measurement unit 2 is used as the input data, and it is inferred whether the target culture A becomes culture B, culture C, or non-intended target cells as the culture state when the target culture A is cultured, from the input data on the basis of a feature amount extraction model; however, the invention is not limited to this. As the culture state obtained when the target culture A is cultured, for example, identification values (straight lines, curved lines, or the like) that specify culture B and culture C in the state inference space of the feature amount extraction model are set, while identification values that further subdivide the non-intended target cells are specified, and an inference result obtained by distinguishing and inferring a possibility that the target culture A may be induced to differentiate into dead cells (cells in a low-quality state) and the like and other cells among the non-intended target cells, may be obtained on the basis of the feature amount extraction model.

As described above, a feature amount extraction model in which an inference result that distinguishes a possibility of inducing differentiation into dead cells (cells in a low-quality state) and the like and other cells among the non-intended target cells, is obtained from a measurement result (input data) of the target culture A by the measurement unit 2, may be generated by supervised learning or unsupervised learning, in the same manner as the case of the above-mentioned culture B, culture C, and the like.

In the above-described fourth embodiment, when the culture state obtainable at the time of culturing the culture A is inferred from the measurement result of the target culture A obtained by the measurement unit 2 on the basis of a feature amount extraction model, for example, the future culture state of the target culture A (culture B, culture C, and non-intended target cells) may be inferred while performing detection of an abnormality in the target culture A in the state inference space of the feature amount extraction model on the basis of a local outlier factor method or the like.

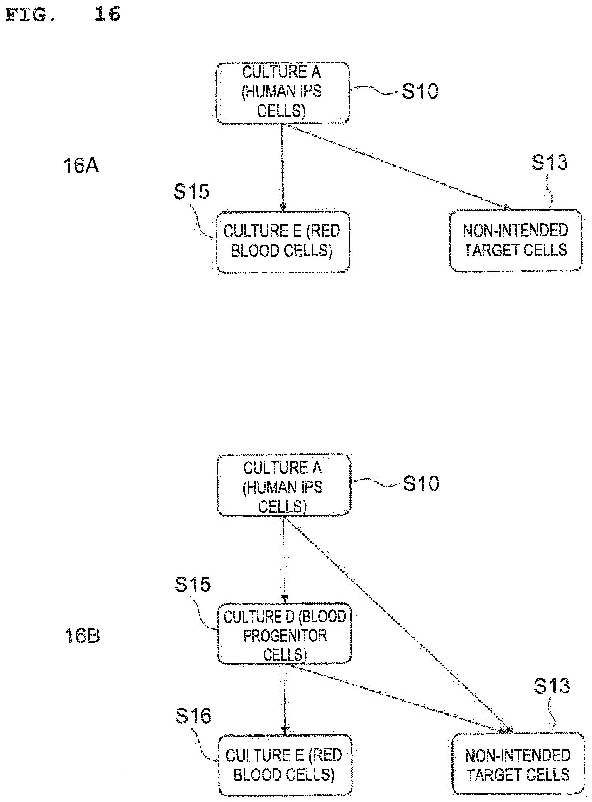

In the above-described fourth embodiment, as shown in FIG. 13, a case in which, when the culture A is cultured, the culture A is induced to differentiate into any of three types such as culture B, culture C, and non-intended target cells, has been described; however, the invention is not limited to this. For example, as shown in 16A in FIG. 16, the invention may also be applied to a case in which, when the culture A is cultured (step S10), the culture A is induced to differentiate into culture E (for example, red blood cells) as shown in step S15, or non-intended target cells (various blood cells such as red blood cells, white blood cells, and platelets) as shown in step S13, as well as a case in which the culture A is induced to differentiate into four or more types of cells.

In addition to that, for example, as shown in 16B in FIG. 16, the state inference system 1d according to the fourth embodiment may also be applied to a case in which, when the culture A is cultured, the culture A is induced to differentiate in multiple stages into culture E through culture D. In this case, an example is shown in 16B in FIG. 16, in which, when culture A (for example, human iPS cells (iPS cells) shown in step S10 is cultured in a culture medium including a predetermined cytokine, depending on the influence on the state of the culture A or the like, the culture A reaches one of two ways of culture states, that is, the culture A is induced to differentiate into culture D (step S15), which is blood progenitor cells, or the culture A becomes non-intended target cells (step S13) such as other different cells, dead cells, or cells in a low-quality state. Furthermore, in this example, an example is described in which, when the obtained culture D is further cultured thereafter, the culture D reaches one of two ways of culture states, that is, the culture D is induced to differentiate into culture E (red blood cells), or the culture D becomes non-intended target cells (step S13) such as other different cells, dead cells, or cells in a low-quality state.

In such a case, in the state inference system 1d, for example, a first feature amount extraction model that infers whether culture D will be obtained by culturing the culture A, or whether non-intended target cells will be obtained by culturing the culture A, is generated by the state inference unit 10a. Furthermore, in the state inference system 1d, a second feature amount extraction model that infers whether culture E will be obtained by culturing the culture D, or whether non-intended target cells will be obtained by culturing the culture D, is generated by the state inference unit 10a.

Since the generation of a feature amount extraction model as described herein is carried out in the same manner as described above, the description thereof will not be repeated here. In addition, as the feature amount extraction model, as described above, a feature amount extraction model for culture D, which infers whether culture D will be obtained by culturing the culture A, a feature amount extraction model that infers whether non-intended target cells will be obtained by culturing the culture A, a feature amount extraction model for culture E, which infers whether culture E will be obtained by culturing the culture D, and a feature amount extraction model that infers whether non-intended target cells will be obtained by culturing the culture D, may be generated each separately.

The measurement unit 2, which is a flow cytometer, measures the characteristic values of each of a plurality of target cultures A and obtains the obtained measurement results as analytical data. The state inference unit 10a inputs the analytical data of the target culture A into the first feature amount extraction model and obtains an inference result that infers whether the culture state of the target culture A will be culture D or non-intended target cells in the future when culturing of the target culture A is continued as it is, on the basis of the first feature amount extraction model.

When the state inference unit 10a obtains an inference result that infers the future culture state for each target culture A, the state inference unit 10a outputs this to the next step decision unit 22. The next step decision unit 22 decides on a corresponding processing step between a processing step of culturing the target culture A under optimal culturing conditions for inducing differentiation of the target culture A into culture D (hereinafter, also referred to as a processing step for culture D) and a processing step of discarding the target culture A (hereinafter, also referred to as a first disposal processing step) on the basis of the inference result for the target culture A, and outputs the decision to the sorting unit 25 as the information on next step.

The sorting unit 25 automatically sorts the target culture A on the basis of the information on next step received from the next step decision unit 22 and selects and sends out only the target culture A for which the information on next step of the processing step for culture D has been obtained to the first culturing unit 26a. The sorting unit 25 sends out the target culture A for which the information on next step of the first disposal processing step has been obtained on the basis of an inference result that the target culture A will become non-intended target cells, to the disposal unit 27, and the target culture A is discarded by the disposal unit 27.

Next, the measurement unit 2, which is a flow cytometer, takes a culture D obtained by culturing the target culture A in the first culturing unit 26a as the target culture D, and an inference result obtained by measuring the characteristic values of each target culture D is obtained as analytical data. The state inference unit 10a inputs the analytical data of each target culture D into the second feature amount extraction model, and infers whether the future culture state of the target culture D becomes culture E or non-intended target cells when culturing of the target culture D is continued as it is, on the basis of the second feature amount extraction model.

When the state inference unit 10a obtains an inference result that infers the future culture state for each target culture D, the state inference unit 10a outputs this to the next step decision unit 22. The next step decision unit 22 decides on a corresponding processing step between a processing step of culturing the target culture D under optimal culturing conditions for inducing differentiation of the target culture D into culture E (hereinafter, also referred to as a processing step for culture E) and a processing step of discarding the target culture D (hereinafter, also referred to as a second disposal processing step) on the basis of the inference result for the target culture D, and outputs the decision to the sorting unit 25 as the information on next step.

The sorting unit 25 automatically sorts the target culture D on the basis of the information on next step received from the next step decision unit 22 and selects and sends out only the target culture D for which the information on next step of the processing step for culture E has been obtained to the second culturing unit 26b. The sorting unit 25 sends out the target culture D for which the information on next step of the second disposal processing step has been obtained on the basis of an inference result that the target culture D will become non-intended target cells, to the disposal unit 27, and the target culture D is discarded by the disposal unit 27.

### (4-3) Others

In the state inference systems 1a, 1c, and 1d according to the above-described first embodiment to fourth embodiment, the culture state of a culture may be inferred from the input data obtained in the measurement unit on the basis of a feature amount extraction model, in order to breed the culture.

In this case, for example, in the example shown in 16B in FIG. 16, a target culture A for which an inference result that the target culture A becomes culture D by the state inference step is sorted (sorting step), the target culture A is cultured in the first culturing unit to obtain culture D (culturing step), and culture D' (not shown in the diagram) is obtained by adding a mutagen that induces mutation or applying genetic manipulation, to the obtained culture D (mutation step). Then, a feature amount extraction model that infers the future culture state of the culture D' is generated, and the culture state of the culture D' is inferred by using the feature amount extraction model (state inference step). In this way, in the state inference systems 1a, 1c, and 1d, it is possible to obtain a culture that has been modified to have new functions and the like by keeping on selection and mutation of the culture state of the culture by repeating the state inference step, the sorting step, the culturing step, and the mutation step, and culturing the selected culture (for example, a culture group consisting only of mutated cultures, or a culture group in a state including both mutated cultures and non-mutated cultures), or it is also possible to carry out breeding of the cultures.

With regard to the above-described breeding, it is not necessarily essential to carry out the above-described mutation step, for example, several kinds of target cultures A for which the future culture state has been inferred on the basis of an inference result of a feature amount extraction model may be sorted, and the above-described culturing step, state inference step, sorting step, and the like may be repeated, according to a known breeding process. In addition, as other breeding, culture D' ' and the like with mutations obtained by culturing a target culture A, for which the culture state has been inferred on the basis of an inference result of a feature amount extraction model, may be sorted, and the above-described culturing step, state inference step, and sorting step may be repeated. Furthermore, a target culture A that may reach a mutant culture state in the future on the basis of an inference result of a feature amount extraction model, may be sorted, and the above-described culturing step, state inference step, sorting step, and the like may be repeated.

In the above-described fourth embodiment, a case in which a flow cytometer is applied as the measurement unit, and analytical data obtainable from the flow cytometer are used as the input data, has been described; however, the invention is not limited to this, and for example, a mass spectrometer, a microscope apparatus, a Raman spectrometer, chromatography, a digital PCR measurement apparatus, a nuclear magnetic resonance apparatus, an antibody quantification kit, and a nucleic acid sequencing apparatus may be applied as the measurement unit. In this case, as the input data, a mass spectrum obtained from a mass spectrometer, image data obtained from a microscope apparatus, a Raman spectrum obtained from a Raman spectrometer, a chromatogram obtained from chromatography, the amount of a specific substance obtained from a digital PCR measurement apparatus, analytical data obtained from a nuclear magnetic resonance apparatus, the amount of a specific antigen obtained from an antibody quantification kit, and gene sequence analysis data obtained from a nucleic acid sequencing apparatus, can be applied as the input data.

In the above-described fourth embodiment, a case in which, as the input data including at least the characteristic values of a plurality of cultures acquired through the measurement unit 2, analytical data of a plurality of cultures acquired by using a flow cytometer are used as the input data, and a feature amount extraction model is generated by using the input data, or an inference result is obtained by a feature amount extraction model, has been described; however, the invention is not limited to this. For example, in addition to the analytical data of a plurality of cultures acquired by using a flow cytometer, a feature amount extraction model may be generated by using input data including parameters (culture parameters) related to culturing conditions such as the culture temperature and culture time of a culturing process for the cultures, the pH of the culture medium, and the stirring speed of the culture medium, or an inference result may be obtained by using a feature amount extraction model.

Similarly to the above-described cases where a mass spectrum obtained from a mass spectrometer, image data obtained from a microscope apparatus, a Raman spectrum obtained from a Raman spectrometer, a chromatogram obtained from chromatography, the amount of a specific substance obtained from a digital PCR measurement apparatus, analytical data obtained from a nuclear magnetic resonance apparatus, the amount of a specific antigen obtained from an antibody quantification kit, and gene sequence analysis data obtained from a nucleic acid sequencing apparatus, are used as the input data, input data including parameters related to the culturing conditions (culture parameters) may also be employed in addition to those data, and a feature amount extraction model may be generated by using the input data, or an inference result may be obtained by using a feature amount extraction model.

### Reference Signs List

- 1a, 1c, 1d:: state inference system
- 2:: measurement unit
- 10a, 10c:: state inference unit
- 12:: determination unit
- 14:: measurement target optimization inference unit
- 15:: culture parameter optimization inference unit

## Claims

1. A state inference system comprising:
a measurement unit for using any of a culture medium, a culture cultured by using the culture medium, a culture medium extract extracted from the culture medium, and a culture extract extracted from the culture, as a biological sample and a target biological sample, and measuring characteristics of a plurality of biological samples including the biological sample at a certain time point and characteristic of the target biological sample being the same type as the biological sample at the certain time point;
a database for storing a feature amount extraction model that characterizes a distribution of input data including at least characteristic values of the plurality of biological samples acquired through the measurement unit; and
a state inference unit for inferring a culture state of the culture or inferring a culture time during which the culture reaches a predetermined culture state, from the input data of the target biological sample on the basis of the feature amount extraction model.

2. The state inference system according to claim 1,
wherein the measurement unit measures characteristics of the plurality of biological samples and the target biological sample before a culturing process is started.

3. The state inference system according to claim 1,
wherein the measurement unit measures characteristics of the plurality of biological samples and the target biological sample in the middle of being used in a culturing process.

4. The state inference system according to any one of claims 1 to 3,
wherein the state inference unit infers a culture state of the culture for which a culture period determined in a culturing process has elapsed, and culturing has been ended.

5. The state inference system according to any one of claims 1 to 3,
wherein the state inference unit infers a culture state of the culture in the middle of culturing, which is being cultured by a culturing process.

6. The state inference system according to any one of claims 1 to 5,
wherein the measurement unit measures characteristics of the plurality of biological samples and the target biological sample in the middle of being used in a culturing process along a timeline, and
the state inference unit infers a culture state of the culture or infers the culture time, on the basis of the feature amount extraction model, in which the characteristic values are the input data and a distribution of the characteristic values of the plurality of biological samples are characterized at each measurement time for the characteristic values, from the characteristic values of the target biological sample at each measurement time for the characteristic values.

7. The state inference system according to any one of claims 1 to 6, further comprising:
a determination unit for determining a state of the target biological sample on the basis of an inference result of the culture state or an inference result of the culture time obtained by the state inference unit.

8. The state inference system according to claim 7,
wherein the determination unit determines the presence or absence of an abnormality of the target biological sample on the basis of an inference result of the culture state or an inference result of the culture time obtained by the state inference unit.

9. The state inference system according to any one of claims 1 to 5,
wherein each of the plurality of biological samples and the target biological sample is the culture medium, and
the state inference system further comprises:
a measurement target optimization inference unit for inferring an additive to be added to the culture medium on the basis of an inference result of the culture state or an inference result of the culture time obtained by the state inference unit.

10. The state inference system according to claim 9,
wherein the measurement target optimization inference unit calculates a degree of similarity between a principal component vector defined in a state inference space for the feature amount extraction model and each additive measurement vector obtained by measuring each of a plurality of types of the additives by using the measurement unit, and infers the additive with the additive measurement vector having a high degree of similarity with the principal component vector.

11. The state inference system according to any one of claims 1 to 5,
wherein the state inference unit infers the culture state or infers the culture time, from the input data of the target biological sample on the basis of the feature amount extraction model that uses the characteristic values of the plurality of biological samples and culture parameters related to culturing conditions of a culturing process using the biological samples, as the input data, and characterizes distributions of the characteristic values and the culture parameters.

12. The state inference system according to claim 11, further comprising:
a determination unit for determining appropriateness of the culture parameters on the basis of an inference result of the culture state or an inference result of the culture time obtained by the state inference unit.

13. The state inference system according to any one of claims 1 to 12,
wherein the measurement unit is any one or more selected from a mass spectrometer, a microscope apparatus, a Raman spectrometer, chromatography, a digital PCR measurement apparatus, a nuclear magnetic resonance apparatus, an antibody quantification kit, a nucleic acid sequencing apparatus, and a flow cytometer, and
the characteristic values measured by the measurement unit are a mass spectrum obtained from the mass spectrometer, image data obtained from the microscope apparatus, a Raman spectrum obtained from the Raman spectrometer, a chromatogram obtained from the chromatography, the amount of a specific substance obtained from the digital PCR measurement apparatus, analytical data obtained from the nuclear magnetic resonance apparatus, the amount of a specific antigen obtained from the antibody quantification kit, gene sequence analysis data obtained from the nucleic acid sequencing apparatus, or analytical data obtained from a flow cytometer.

14. The state inference system according to any one of claims 1 to 12,
wherein the measurement unit is an impedance measurement apparatus, and
the characteristic values measured by the measurement unit are impedances of the plurality of biological samples and the target biological sample.

15. The state inference system according to any one of claims 1 to 14,
wherein the culture state of the culture is a yield of the culture, a quality of the culture, a yield of the culture medium extract, or a quality of the culture extract.

16. The state inference system according to any one of claims 1 to 15,
wherein the measurement unit measures characteristics of the culture at the certain time point, and
the state inference unit uses measurement results obtained from the measurement unit as the input data, and infers which culture state among a plurality of types of culture states the culture will reach by culturing, from the input data on the basis of the feature amount extraction model.

17. The state inference system according to claim 16, further comprising:
a next step determination unit for determining a next processing step for the culture according to the type of a culture state of the culture inferred by the state inference unit.

18. The state inference system according to any one of claims 1 to 17,
wherein the state inference unit infers a culture state of the culture from the input data on the basis of the feature amount extraction model in order to breed the culture.

19. A state inference method comprising:
a measurement step of using any of a culture medium, a culture cultured by using the culture medium, a culture medium extract extracted from the culture medium, and a culture extract extracted from the culture, as a biological sample and a target biological sample, and measuring characteristics of a plurality of biological samples including the biological sample at a certain time point and characteristic of the target biological sample being the same type as the biological sample at the certain time point, in a measurement unit;
a storing step of storing, in a database, a feature amount extraction model that characterizes a distribution of input data including at least characteristic values of the plurality of biological samples acquired through the measurement unit; and
a state inference step of inferring a culture state of the culture by using a state inference unit or inferring a culture time during which the culture reaches a predetermined culture state by using a state inference unit, from the input data of the target biological sample on the basis of the feature amount extraction model.
